# EUROPEAN PATENT APPLICATION

(11) **EP 1 790 724 A1**
(43) Date of publication of application: **30.05.2007**
(21) Application number: 05770322.5
(22) Date of filing: 09.08.2005
(51) Int. Cl.: C12N 15/12, C07K 14/47, A01K 67/027, C07K 16/18, C12Q 1/68, C12N 5/10, G01N 33/15, G01N 33/50, G01N 33/68

(54) **KNOCKOUT NONHUMAN ANIMAL**

(30) Priority: 10.08.2004 JP 2004233089
(71) Applicant: Sumitomo Chemical Company, Limited, Tokyo 104-8260 (JP); Dainippon Sumitomo Pharma Co., Ltd., Osaka 541-8524 (JP); Trans Genic Inc., Kamimashiki-gun, Kumamoto 861-2202 (JP)
(72) Inventor: NAITO, Yoshikazu, Toyonaka-shi, Osaka 560-0021 (JP); OEDA, Kenji, 251-1-202, Kamigoryokamie-cho, Kyoto-shi, Kyoto 603-8147 (JP); SUKATA, Tokuo, Sumitomo Chemical Co. Ltd., Osaka-shi, Osaka 554-8558 (JP); INOUE, Yoshifumi, Suita-shi, Osaka 564-0053 (JP)
(74) Representative: Vossius & Partner
(86) International application number: PCT/JP2005/014881
(87) International publication number: WO 2006/016692

(57) **Abstract**

The present invention relates to a knockout non-human animal whose genome comprises a disruption in at least one allele of the PHF3 gene, useful in developing drugs for diseases with the onset of atopic dermatitis or the like; a totipotent cell such as an ES cell which is essential to production of the above non-human animal; use thereof; and so on.

## Description

### Technical Field

The present invention relates to a knockout non-human animal in which the PHF3 gene is disrupted and the like.

### Background Art

Allergic diseases including atopic dermatitis, asthma, allergic rhinitis, allergic conjunctivitis and the like cause hypersensitive responses to an environmental antigen and the like to which normal people do not respond, and lead to failure of each organ via an immunoglobulin E mediated inflammatory mechanism.

There are a variety of genome regions involved in the development of allergic diseases, and there are many disease-causative genes corresponding to the genome regions (Halapi et al., Curr Opin Pulm Med., 10:22-30, 2004; Lee et al., Nat Genet., 26:470-473, 2000; Cookson et al., Lancet, 1:1292-1295, 1989; Bradley et al., Hum. Mol. Genet., 11;1539-1548, 2002). In some of genome regions involved in allergic diseases, a disease-causative gene and its physiological mechanism have been known. For example, it has been known that the genome region 5q31 encodes the cytokine IL-4 gene and mutation of said gene is associated with severity of atopic dermatitis ( Bradley et al., Hum. Mol. Genet., 11;1539-1548, 2002; Novak et al., J. Invest. Dermatol., 119:870-875, 2002). Secretion of so-called Th2 cytokines such as IL-4, 5, 13 and the like is promoted by activation of helper T2 cells. These cytokines stimulate B cells to induce an allergic reaction accompanied by an increased expression of immunoglobulin E.

Among model animals for atopic dermatitis, a NC/Nga mouse, a NOA mouse, a C57BL/Ka cpdm/cpdm mouse and a Ds-Nh mouse draw attention as spontaneous atopic dermatitis model animals (Atsuko Itakura et al., SAISHIN IGAKU, vol.53: 2848-2854, 1998; Mouse Genome, 95:698-700, 1997; HogenEsch et al., Am J Pathol., 143:972-982, 1993; Hikita et al., J Dermatol Sci., 30:142-153, 2002). In these model animals, mutant genes have not been identified. In addition, a TNP-IgE transgenic mouse, a IL-18 transgenic mouse, a Caspase-1 transgenic mouse and a cathepsin E knockout mouse which are produced by gene alteration are also known as model animals for atopic dermatitis (Matsuoka et al., Int Immunol., 11:987-994, 1999; Yoshimoto et al., Nat Immunol., 1:132-137, 2000; Yamanaka et al., J Immunol., 165:997-1003, 2000; Tsukuba et al., J Biochem (Tokyo)., 134:893-902, 2003).

The gene of the PHF3 protein has been cloned as a gene whose expression is reduced in human gliomas (Fischer et al., Cytogenet Cell Genet., 94 : 131-136, 2001). The protein is characterized by possession of a PHD domain (a type of zinc finger domain) and therefore has been called PHD finger domain 3.

### Disclosure of Invention

An object of the present invention is to provide a knockout non-human animal and the like in which a novel causative gene is disrupted, useful in developing remedies and the like for diseases with the development of atopic dermatitis.

The present inventors disrupted the PHF3 gene on a chromosome of a mouse ES cell, mixed the resulting ES cell with the blastocyst of a parent animal, allowed embryonic differentiation in the parent animal to continue until a baby was born, and thereby a chimeric primitive mouse was produced as an offspring. Then, the present inventors bred the chimeric primitive mouse to produce a progeny (hereinafter, the chimeric primitive mouse and a progeny thereof are referred to as the present knockout mouse in some cases). In the present knockout mouse, scab formation and cell infiltration into the skin dermis layer were high-frequently observed, and the total immunoglobulin E level in blood was extremely high. Based on these findings, the present inventors found that said animal can be a model mouse representing disease states similar to the development of atopic dermatitis, and as a result, completed the present invention.

That is, the present invention provides:
1. A knockout non-human animal whose genome comprises a disruption in at least one allele of the PHF3 gene (hereinafter, referred to as the present non-human animal in some cases);
2. The knockout non-human animal according to the above item 1, wherein the disruption leads to an insufficient expression of the PHF3 gene;
3. The knockout non-human animal according to the above item 1 or 2, wherein the disruption leads to the development of atopic dermatitis;
4. The knockout non-human animal according to any one of the above items 1 to 3, wherein the disruption prevents transcription of a full-length mRNA from the allele of the PHF3 gene;
5. The knockout non-human animal according to any one of the above items 1 to 4, wherein the disruption prevents transcription from exon 3 and the downstream region thereof in the allele of the PHF3 gene;
6. The knockout non-human animal according to any one of the above items 1 to 5, wherein the disruption comprises insertion of a gene cassette;
7. The knockout non-human animal according to the above item 6, wherein the gene cassette comprises a nucleotide sequence encoding a selectable marker;
8. The knockout non-human animal according to the above item 6, wherein the gene cassette comprises a nucleotide sequence encoding a selectable marker and a transcription termination signal;
9. The knockout non-human animal according to any one of the above items 1 to 8, wherein the disruption is a homozygous disruption;
10. The knockout non-human animal according to any one of the above items 1 to 9, wherein the non-human animal is a mammal;
11. The knockout non-human animal according to any one of the above items 1 to 9, wherein the non-human animal is a rodent;
12. The knockout non-human animal according to any one of the above items 1 to 9, wherein the non-human animal is a mouse;
13. The knockout non-human animal according to the above item 12, wherein the PHF3 gene comprises a nucleotide sequence encoding the amino acid sequence of SEQ ID NO: 1;
14. The knockout non-human animal according to any one of the above items 1 to 13, wherein the disruption leads to representation of one or more properties selected from the group consisting of the following (a) to (d):
   (a) high-frequency formation of scabs on the skin as compared with the wild-type non-human animal;
   (b) high-frequency cell infiltration into the dermis layer of the skin as compared with the wild-type non-human animal;
   (c) a high level of total immunoglobulin E in the blood as compared with the wild-type non-human animal; and
   (d) a variation in the expression amount of an atopic dermatitis-associated factor as compared with the wild-type non-human animal;
15. A tissue or cell isolated from the knockout non-human animal according to any one of above items 1 to 14;
16. The tissue or cell according to the above item 15, which is a T cell, a B cell or a leukocyte-derived cell;
17. A cultured animal cell whose genome comprises a disruption in at least one allele of the PHF3 gene;
18. The cultured animal cell according to the above item 17, wherein the disruption leads to an insufficient expression of the PHF3 gene;
19. The cultured animal cell according to the above item 17 or 18, which is totipotent;
20. The cultured animal cell according to any one of the above items 17 to 19, wherein the PHF3 gene comprises a nucleotide sequence encoding the amino acid sequence of SEQ ID NO: 1;
21. The cultured animal cell according to the above item 17 or 18, wherein the PHF3 gene comprises a nucleotide sequence encoding the amino acid sequence of SEQ ID NO: 7;
22. An animal model for a disease with the development of atopic dermatitis, comprising the non-human animal according to any one of the above items 1 to 14;
23. A method for analyzing a state of a disease associated with the PHF3 protein, which comprises performing on the non-human animal according to any one of the above items 1 to 14 during the period from the embryonic stage to death: observation of growth and differentiation, development, and living behavior; a histopathological test; or a biochemical test;
24. The analyzing method according to the above item 23, wherein the disease associated with the PHF3 protein is a disease with the development of atopic dermatitis;
25. A method of assessing an ability to control atopic dermatitis, which comprises:
   (1) the first step of contacting the non-human animal according to any one of the above items 1 to 14 or a part thereof with a test substance,
   (2) the second step of measuring an expression amount of an atopic dermatitis-associated factor in the non-human animal or a part thereof which has been contacted with the test substance in the first step, or an index value correlated with the expression amount, and then comparing the measured value with a control, and
   (3) the third step of assessing an ability of the test substance to control atopic dermatitis on the basis of the comparison result of (2);
26. The assessing method according to the above item 25, wherein the ability to control atopic dermatitis is an ability to control the expression of an atopic dermatitis-associated factor;
27. The assessing method according to the above item 25 or 26, wherein the expression amount of an atopic dermatitis-associated factor is a RNA amount or a protein amount of an atopic dermatitis-associated factor;
28. The assessing method according to any one of the above items 25 to 27, wherein the atopic dermatitis associated factor is one or more factors selected from the group consisting of IFN-γ, IL-4, IL-5, IL-13, IL-18 and immunoglobulin E;
29. The assessing method according to the above item 28, wherein the atopic dermatitis-associated factor is immunoglobulin E;
30. The assessing method according to any one of the above items 25 to 28, wherein the index value correlated with the expression amount of an atopic dermatitis-associated factor is an amount of formed scabs;
31. The assessing method according to any one of the above items 25 to 28, wherein the index value correlated with the expression amount of an atopic dermatitis-associated factor is a cell infiltration amount into the dermis layer of the skin;
32. The assessing method according to any one of the above items 25 to 28, wherein the index value correlated with the expression amount of an atopic dermatitis-associated factor is the total immunoglobulin E level in the blood;
33. A method of screening an atopoic dermatitis controlling substance, which comprises selecting a test substance having an ability to control atopic dermatitis on the basis of an ability to control atopic dermatitis assessed by the assessing method according to any one of the above items 25 to 32;
34. A remedy or prophylactic for a disease with the development of atopic dermatitis, which comprises a substance having an ability to control atopic dermatitis obtained by the screening method according to the above item 33 as an active ingredient;
35. A recombinant vector for producing the non-human animal according to any one of the above items 1 to 14 or the cell according to any one of the above items 17 to 21, wherein the vector carries a gene cassette comprising a selectable marker gene from which a promoter region has been removed, and wherein the vector further comprises a nucleotide sequence homologous to a part of the nucleotide sequence of the PHF3 gene;
36. The recombinant vector according to the above item 35, wherein the gene cassette is a gene cassette in which mouse EN2-derived SA (splicing acceptor), encephalomyocarditis Virus-derived IRES (internal ribosome entry site), βgeo in which β-galactosidase and a neomycin resistant gene are fused, and SV40-derived pA (polyadenylation signal) are connected;
37. Use of a substance selected from:
   (1) the PHF3 protein or a partial region thereof;
   (2) a polynucleotide comprising a nucleotide sequence encoding the amino acid sequence of the protein or a partial region thereof described in (1), or a nucleotide sequence complementary to the nucleotide sequence;
   (3) a DNA encoding a RNA having a ribozyme activity which specifically cleaves a nucleotide sequence encoding the amino acid sequence of the protein or a partial region thereof described in (1); and
   (4) a DNA encoding a RNA which suppresses the expression of the PHF3 gene on the basis of RNAi effect when expressed in a cell, for protein therapy or gene therapy of an allergy disease;
38. The use according to the above item 37, wherein the PHF3 protein has an activity for ameliorating allergy disease symptoms and comprises any one of the following amino acid sequences:
   <Amino acid sequence>
   (a) the amino acid sequence of SEQ ID NO: 1 or 7;
   (b) the amino acid sequence of SEQ ID NO: 1 or 7 in which one or more amino acids are deleted, added or substituted;
   (c) an amino acid sequence with 75% or more sequence identity to the amino acid sequence of SEQ ID NO: 1 or 7;
   (d) the amino acid sequence encoded by the nucleotide sequence of SEQ ID NO: 2 or 8;
   (e) an amino acid sequence encoded by a DNA comprising a nucleotide sequence with 80% or more sequence identity to the nucleotide sequence of SEQ ID NO: 2 or 8; and
   (f) an amino acid sequence encoded by a DNA which hybridizes with a DNA comprising a nucleotide sequence complementary to the nucleotide sequence of SEQ ID NO: 2 or 8 under stringent conditions;
39. An antibody immunologically specific for a protein comprising the amino acid sequence of SEQ ID NO: 1 or 7 or a partial sequence thereof;
40. Use of a substance selected from:
   (1) the PHF3 protein or a partial region thereof;
   (2) a polynucleotide comprising a nucleotide sequence encoding the amino acid sequence of the protein or a partial region thereof described in (1), or a nucleotide sequence complementary to the nucleotide sequence; and
   (3) a gene of the PHF3 protein, for diagnosing atopic dermatitis;
41. The use according to the above item 40, wherein the PHF3 protein has an activity for ameliorating allergy disease symptoms and comprises any one of the following amino acid sequences:
   <Amino acid sequence>
   (a) the amino acid sequence of SEQ ID NO: 1 or 7;
   (b) the amino acid sequence of SEQ ID NO: 1 or 7 in which one or more amino acids are deleted, added or substituted;
   (c) an amino acid sequence with 75% or more sequence identity to the amino acid sequence of SEQ ID NO: 1 or 7;
   (d) the amino acid sequence encoded by the nucleotide sequence of SEQ ID NO: 2 or 8;
   (e) an amino acid sequence encoded by a DNA comprising a nucleotide sequence with 80% or more sequence identity to the nucleotide sequence of SEQ ID NO: 2 or 8; and
   (f) an amino acid sequence encoded by a DNA which hybridizes with a DNA comprising a nucleotide sequence complementary to the nucleotide sequence of SEQ ID NO: 2 or 8 under stringent conditions; and the like.

### Brief Description of Drawings

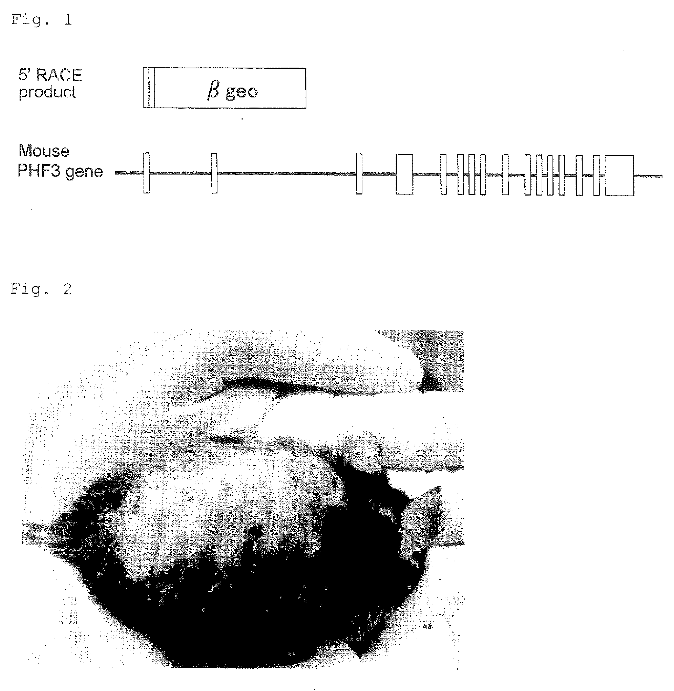

Fig. 1 shows results of decoding of the nucleotide sequence of a trapped gene by a 5' RACE method, using an ES cell used for producing a PHF3 gene knockout mouse. The decoded nucleotide sequence corresponded to the first exon and the second exon of the PHF3 gene.
Fig. 2 is a photograph of the appearance of a mouse with a homozygous knockout PHF3 gene. Hair loss and scab formation were observed over a wide region of the back.
Fig. 3 shows a stained image of tissues (abdominal) such as a skin and a subcutaneous muscle by HE-staining. (a) is a stained image of a thin section of a skin tissue from a normal mouse, (b) is a stained image of a thin section of a subcutaneous muscle tissue from a normal mouse, (c) is a stained image of a thin section of a skin tissue from the present knockout mouse, and (d) is a stained image of a thin section of a subcutaneous muscle tissue from the present knockout mouse. In the case of the present knockout mouse, proliferation of prickle cells, formation of scabs, and cell infiltration into the skin dermis layer were observed and, additionally, cell infiltration into a subcutaneous tissue and atrophy of cutaneous muscle were observed.
Fig. 4 shows results of measurement of the total immunoglobulin E levels in sera of PHF3 gene knockout mice (PHF3 homo KO) and wild-type mice (WK). In the case of the PHF3 gene homo-knockout mice, measured values of respective individuals are shown as bar graphs. In the case of the wide-type mice, the average of measured values of four individuals is shown as a bar graph, and the standard deviation is represented by an error line.
Fig. 5 shows results of the expression amount of PHF3 mRNA investigated in various human tissues. The upper column shows results of amplification of the PHF3 gene by a PCR method. The lower column shows results of amplification of the β actin gene by a PCR method. Of leukocyte samples, the "resting" shows cDNAs derived from hemocytes which were subjected to only a treatment of separation from blood, and the "activated" shows cDNAs derived from a mononuclear cell which was treated with concanavalin A (ConA) and pokeweed mitogen (PWM), a helper T cell which was treated with ConA, a killer T cell which was treated with phytohemagglutinin, and a B cell which was treated with PWM.

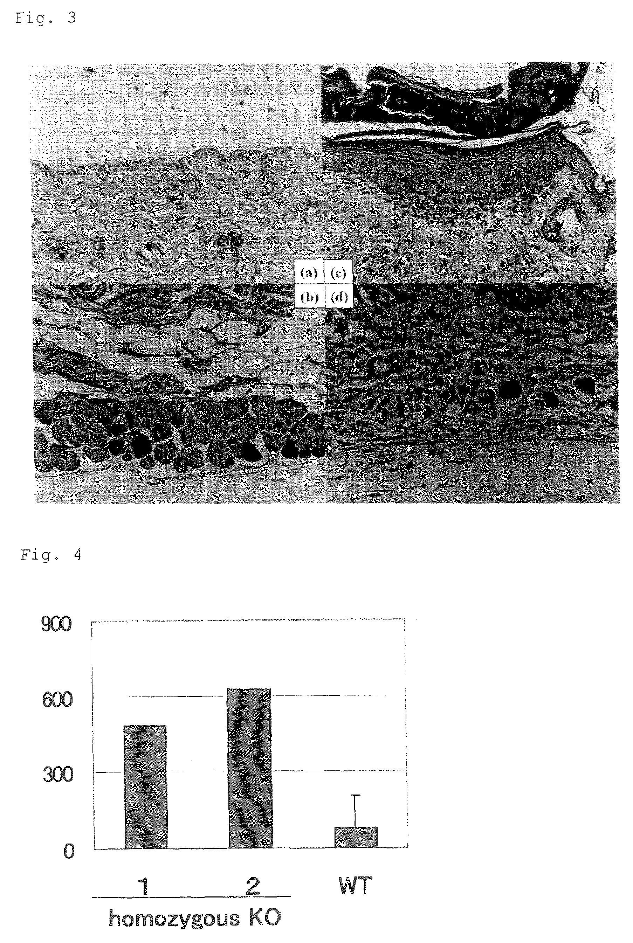

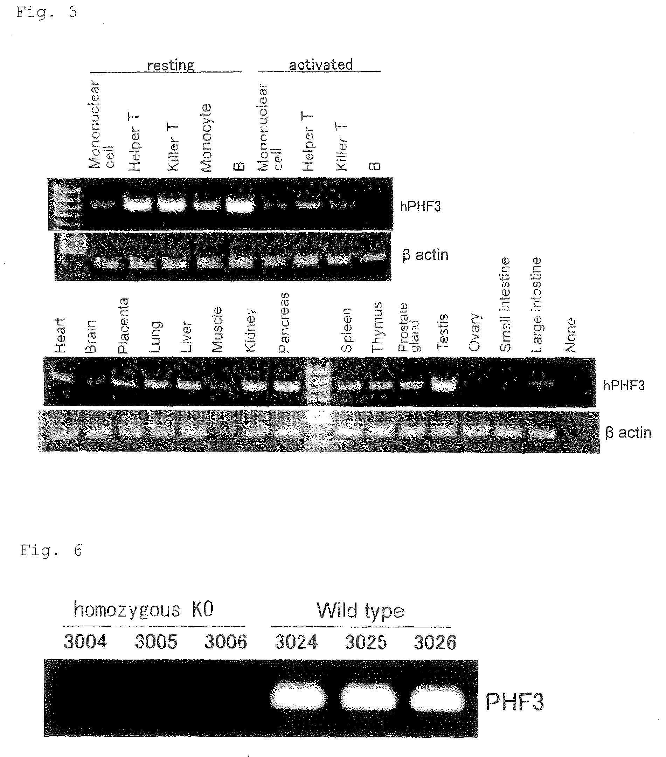

Fig. 6 shows analysis results of expression of the PHF3 gene in the lymph nodes of PHF3 homo-knockout mice and wide-type mice by a reverse transcription PCR method.

### Best Mode for Carrying Out the Invention

The present invention will be explained in detail below.

The present invention provides a knockout non-human animal whose genome comprises a disruption in at least one allele of the PHF3 gene. Herein, the "disruption in an allele" means that the allele(s) of an endogenous gene such as the PHF3 gene is artificially altered so that the expression of at least a part of a protein encoded by the endogenous gene is lost or reduced. Examples of such disruption include a heterozygous disruption in which one of alleles of an endogenous gene is disrupted, and a homozygous disruption in which both of the alleles of an endogenous gene are disrupted.

The knockout non-human animal in the present invention means a transgenic non-human animal in which all or one of alleles at a locus present in cells including germ line cells in the living body of a non-human animal is disrupted (e.g. disrupted by recombination), so that the original function of the gene is not exerted. In the case of the knockout non-human animal provided according to the present invention, all or one of alleles of the PHF3 gene at a locus on a chromosome in a cell constituting a non-human animal living body is disrupted (e.g. disrupted by recombination), so that the active PHF3 protein is not expressed. In such knockout non-human animal, artificial alteration of allele(s) may result in inhibition of the expression ability of the PHF3 gene or substantial loss or reduction of the activity of the PHF3 protein encoded by the allele(s).

The PHF3 gene in the present invention is a region on a chromosome of a non-human animal which corresponds to a mRNA having a nucleotide sequence encoding the amino acid sequence of the PHF3 protein and may contain introns, and it is not limited to specific structure, for example, a specific nucleotide sequence or specific intron-exon structure.

Examples of the PHF3 protein in the present invention (hereinafter, referred to as the present protein in some cases) include (a) a protein comprising the amino acid sequence of SEQ ID NO: 1, (b) a protein comprising the amino acid sequence of SEQ ID NO: 1 in which one or more amino acids are deleted, added or substituted, and having an activity for ameliorating allergy disease symptoms, (c) a protein comprising an amino acid sequence with 75% or more sequence identity to the amino acid sequence of SEQ ID NO: 1, and having an activity for ameliorating allergy disease symptoms, (d) a protein comprising the amino acid sequence encoded by the nucleotide sequence of SEQ ID NO: 2, (e) a protein comprising an amino acid sequence encoded by a DNA comprising a nucleotide sequence with 80% or more sequence identity to the nucleotide sequence of SEQ ID NO: 2, and having an activity for ameliorating allergy disease symptoms, and (f) a protein comprising an amino acid sequence encoded by a DNA which hybridizes with a DNA comprising a nucleotide sequence complementary to the nucleotide sequence of SEQ ID NO: 2 under stringent conditions, and having an activity for ameliorating allergy disease symptoms.

Herein, the "deletion, addition or substitution of amino acids" described in the (b), and the "amino acid sequence with 75% or more sequence identity" and the "nucleotide sequence with 80% or more sequence identity" described in the (c) and the (e) include naturally occurring mutations due to processing which a protein comprising the amino acid sequence of SEQ ID NO: 1 undergoes in a cell, a spices difference or an individual difference between organisms from which the protein is derived, a difference between tissues from which the protein is derived or the like, artificial amino acid mutations, and the like.

An example of an artificial procedure for the "deletion, addition or substitution of amino acid" described in the (b) (hereinafter, collectively referred to as "alteration of amino acid") comprises introducing a mutation into a DNA encoding the amino acid sequence of SEQ ID NO: 1 by a conventional site-specific mutagenesis method and then expressing said DNA by a conventional method. Herein, examples of the site-specific mutagenesis method include a method utilizing amber mutation (gapped duplex method, Nucleic Acids Res., 12, 9441-9456(1986)), a PCR method using primers for introducing a mutation, and the like.

The number of amino acids to be altered as described above is at least one residue, specifically 1 or a few, or more. The number of such alteration may be in such a range that an activity for ameliorating allergy disease symptoms can be found in the present protein.

Among the aforementioned deletion, addition and substitution, alteration involving substitution of amino acid is particularly preferred. More preferably, the substitution is substitution with amino acid whose properties such as hydrophobicity, charge, pK, a steric structural characteristic and the like are similar. Examples of such substitution include substitution within the groups of i) glycine, alanine; ii) valine, isoleucine, leucine; iii) aspartic acid, glutamic acid, asparagine, glutamine, iv) serine, threonine; v) lysine, arginine, histidine; vi) phenylalanine, tyrosine.

The "sequence identity" in the present invention refers to identity and homology between two nucleic acid or two protein sequences. The "sequence identity" is determined by comparing two sequences aligned in the optimal state, over the region of a sequence to be compared. Herein, a nucleic acid or protein to be compared may have addition or deletion (e.g. gap etc.) in the optimal alignment of two sequences. Such sequence identity can be calculated by producing alignment utilizing the ClustalW algorism (Nucleic Acid Res., 22(22):4673-4680(1994)), for example, using Vector NTI. The sequence identity is determined using sequence analysis software, specifically, Vector NTI or GENETYX-MAC, or an analysis tool provided in public database. The public database is generally available, for example, in a website (http://www.ddbj.nig.ac.jp) of DNA Data Bank of Japan [an international DNA data bank operated in Center for International Biology and DNA Data Bank of Japan; CIB/DDBJ)] or the like.

The sequence identity in the present invention is 75% or more, preferably 85% or more, more preferably 90% or more at the amino acid level. At the nucleic acid level, the sequence identity is 80% or more, preferably 90% or more, more preferably 95% or more.

Regarding the "hybridizes under stringent conditions" described in the (f), the hybridization can be performed according to a conventional method described in, for example, Sambrook J., Frisch E.F.,Maniatis, T., Molecular Cloning 2nd edition, Cold Spring Harbor Laboratory Press or the like. An example of the "stringent conditions" is hybridization at 45°C in a solution containing 6 x SSC (a solution containing 1.5 M NaCl and Q.15 M trisodium citrate is defined as 10 x SSC) and 50% formamide, followed by washing with 2 x SSC at 50°C (Molecular Biology, John Wiley & Sons, N.Y. (1989), 6.3.1-6.3.6). The salt concentration in a washing step can be selected, for example, from the range of 2 x SSC at 50°C (low stringent condition) to 0.2 x SSC at 50°C (high stringent condition). The temperature in a washing step can be selected, for example, from the range of room temperature (low stringent condition) to 65°C (high stringent condition). In addition, both the salt concentration and the temperature may be changed.

More specific examples of the PHF3 gene in present invention include (I) a gene comprising a nucleotide sequence encoding the amino acid sequence of SEQ ID NO: 1 or 7, (II) a gene comprising the nucleotide sequence of SEQ ID NO: 2 or 8, and the like, and naturally occurring allele gene polymorphism of these genes is also included. These genes can be obtained according to a conventional genetic engineering method (e.g. a method described in Sambrook J., Frisch E.F., Maniatis T., Molecular Cloning 2nd edition, Cold Spring Harbor Laboratory press etc.).

The nucleotide sequence of SEQ ID NO: 2 or 8 is a nucleotide sequence of a cDNA corresponding to a mRNA comprising a nucleotide sequence encoding the amino acid sequence of SEQ ID NO: 1 or 7 (i.e. a nucleotide sequence of a mRNA comprising a nucleotide sequence encoding the amino acid sequence of SEQ ID NO: 1 or 7 in which U is substituted with T), which comprises a nucleotide sequence of an untranslated region at the 5'-terminus and the 3'-terminus. The PHF3 genome gene contains a nucleotide sequence of a cDNA such as the nucleotide sequence of SEQ ID NO: 2, and has structure in which said nucleotide sequence is interrupted by introns, that is, intron-exon structure.

In the case of the nucleotide sequence of SEQ ID NO: 2, a more specific example of the intron-exon structure contains introns at positions between nucleotide numbers 312 and 313 (between exons 1 and 2), between nucleotide numbers 582 and 583 (between exons 2 and 3), between nucleotide numbers 744 and 745 (between exons 3 and 4), between nucleotide numbers 2461 and 2462 (between exons 4 and 5), between nucleotide numbers 2771 and 2772 (between exons 5 and 6), between nucleotide numbers 2955 and 2956, (between exons 6 and 7), between nucleotide numbers 3100 and 3101 (between exons 7 and 8), between nucleotide numbers 3257 and 3258 (between exons 8 and 9), between nucleotide numbers 3374 and 3375 (between exons 9 and 10), between nucleotide numbers 3506 and 3507 (between exons 10 and 11), between nucleotide numbers 3642 and 3643 (between exons 11 and 12), between nucleotide numbers 3838 and 3839 (between exons 12 and 13), between nucleotide numbers 3984 and 3985 (between exons 13 and 14), between nucleotide numbers 4076 and 4077 (between exons 14 and 15), and between nucleotide numbers 4272 and 4273 ((between exons 15 and 16) on the nucleotide sequence of SEQ ID No:2.

Specific examples of the PHF3 gene include the mouse PHF3 gene (SEQ ID NO: 2: GenBank accession No. XM_129836, the corresponding mouse PHF3 protein SEQ ID No: 1: GenBank accession No.XP_129836) and the human PHF3 gene (SEQ ID NO: 8: GenBank accession No. NM_015153, the corresponding human PHF3 protein SEQ ID NO: 7: GenBank accession No. NP_055968). However, the source of the PHF3 gene is not limited to a mouse and a human. Examples of the PHF3 gene also include orthologues of the PHF3 gene derived from a mouse or a human. The sequence identity between the mouse PHF3 gene and the human PHF3 gene is 82.0% at the nucleic acid level and 77.5% at the amino acid level.

Examples of the "activity for ameliorating allergy disease symptoms" as used herein include activities for ameliorating formation of scabs, cell infiltration into the skin dermis layer and a high level of total immunoglobulin E in the blood which are symptoms characteristic of atopic dermatitis. Other examples of the "activity for ameliorating allergy disease symptoms" include activities for ameliorating an abnormal level of a cytokine, an abnormal level of histamine, and the like.

The non-human animal in the present invention is an animal comprising the PHF3 gene other than a human. Examples of the non-human animal include non-human mammals (e.g. rabbit, dog, cat, guinea pig, hamster, mouse, rat, sheep, gout, pig, horse, bovine, monkey etc.). Inter allia, Robentia mammals such as a mouse, a rat, a guinea pig and the like is preferred, and a mouse and a rat are particularly preferred.

A part of the non-human animal in the present invention is not particularly limited as long as it is a tissue or a cell derived from the non-human animal. Examples of a part of the non-human animal include parts of the body including skin tissues such as a skin and a subcutaneous muscle, various fat tissues such as a peritesticular fat tissue, a retroperitoneal fat tissue, a mesenteric fat tissue, a subcutaneous fat tissue and a brown fat tissue, and other tissues such as heart, lung, kidney, gallbladder, liver, pancreas, spleen, intestine, orchis (testis), ovary, uterus, placenta, muscle, blood vessel, brain, marrow, thyroid gland, thymus, mammary gland and lymph node. In addition, body fluid such as blood, lymph fluid and urine derived from the non-human animal is also included in a part of the non-human in the present invention.

Further, examples of a part of the non-human animal include not only cultured cells obtained by isolating and culturing cells contained in the aforementioned tissues, organs or body fluid (including collected first-generation primary cells and cells established from the primary cells) and extracts, but also various organs at the developmental stage in an embryo at the embryonic stage, cultures of cells associated with the organs, and ES cells regardless of the presence or absence of their differentiation ability and proliferation ability.

Specific examples of such a tissue or cell derived from the non-human animal include cell strains derived from T cells, B cells and leukocytes and the like.

The first aspect of the present invention relates to an individual of a knockout non-human animal, a progeny animal thereof, or a part thereof.

The present non-human animal is provided, for example, as a heterozygote or a homozygote in which both or one of alleles of the diploid PHF3 gene on a chromosome in somatic cells is replaced with a function-deficient mutant or function-reduced mutant of the PHF3 gene, or all or one of alleles of the diploid genome gene is disrupted (e.g. disrupted by recombination) so that its function is deleted or reduced.

The present non-human animal can be produced according to a conventional method such as a known target gene recombination method (gene targeting method: e.g. Masahiro Muramatsu, Masa Yamamoto ed., "Experimental Medicine Separate Volume, New Edition, Genetic Engineering Handbook 3rd edition" (published by Yodosha in 1999), p.239-256, Science 244:1288-1292,1989). For example, both or one of alleles of the targeted diploid genome gene (i.e. the genome gene of the PHF3 protein) on a chromosome in ES (embryonic stem) cells of a non-human animal whose PHF3 gene is to be altered is disrupted or recombined, for example using a targeting vector carrying a selectable marker gene (i.e. a DNA comprising a nucleotide sequence encoding a selectable marker) from which a promoter region has been removed (selectable marker gene promoterless recombinant vector), by a homologous recombination method (i.e. promoter trap method) by which a part of the targeted gene on a chromosome is replaced with the targeting vector. After the ES cell is mixed with the blastocyst of a parent animal, embryonic differentiation is allowed to continue until a baby is born, and thereby a chimeric primitive mouse is produced as an offspring. In this method, ES cells and the like are used as totipotent cells. As the totipotent cells, ES cells and the like of a mouse (Nature 292:154-156, 1981), a rat (Dev. Biol. 163 (1): 288-292, 1994), a monkey (Proc. Natl. Acad. Sci. U.S.A. 92(17): 7844-7848, 1995) and a rabbit (Mol. Reprod. Dev. 45(4): 439-443, 1996) have been established. In addition, an EG (embryonic germ) cell of a pig has been established (Biol. Reprod 57(5): 1089-1095, 1997). Therefore, the present non-human animal is preferably produced with these animal species. In particular, a mouse is optimal because technique is ready for production of a knockout animal using a mouse.

A specific procedure for producing the present non-human animal will be explained below. First, a DNA of a genome gene encoding the PHF3 protein is isolated, and then subjected to gene manipulation in vitro to disrupt the genome gene so that the expression of the genome gene becomes insufficient. Examples of a method for such artificial alteration of the PHF3 gene include a method comprising deletion or substitution of a part or all of the nucleotide sequence of the gene by a genetic engineering procedure, a method comprising insertion of other genes or replacement with other genes, and the like. Such alteration results in a frame shift in an open reading frame of codons, insertion of a nucleotide sequence which terminates transcription of a gene (e.g. polyA addition signal etc.) into an intron part between exons, disruption of the function of a promoter or an exon, or the like, so that the expression of the gene can be prevented.

Specifically, for example, a mutated DNA is produced to replace the genome gene with a function-deficient mutant or a function-reduced mutant of the gene, or disrupt or recombine the genome gene so that its function is deleted or reduced.

A DNA of the genome gene is obtained, for example, by screening a genome DNA library of a non-human animal such as a mouse using an oligonucleotide probe synthesized based on a known cDNA nucleotide sequence of the mouse-derived PHF3 gene. Alternatively, a DNA of the objective genome gene can be also obtained by a PCR method using synthetic oligonucleotides corresponding to a part or both ends of the aforementioned cDNA as primers. When a non-human animal other than a mouse is a subject, a DNA of the gene derived from various non-human animals can be isolated by the aforementioned methods using oligonucleotides synthesized based on the nucleotide sequence of the aforementioned cDNA as probes or primers. In addition, based on the nucleotide sequence of the aforementioned cDNA, the structure of the genome gene is analyzed in detail to elucidate intron-exon structure in the genome gene and the nucleotide sequences of introns.

Based on the elucidated information, for example, a DNA containing a part of the genome gene can be altered by replacement, deletion and/or addition so that at least a part of a nucleotide sequence in a protein-encoding region of the gene is changed, to produce a mutated DNA for replacing the genome gene with a function-deficient mutant or a function-reduced mutant of the gene, or disrupting or recombining the genome gene so that its function is deleted or reduced.

The mutated DNA thus prepared by altering a part of a DNA of the PHF3 gene is used to prepare a targeting vector for introducing a mutation into the PHF3 gene in a totipotent cell such as an ES cell according to a known method (e.g. Science 244: 1288-1292, 1989). Herein, the "targeting vector" is a DNA molecule for disrupting or recombining an objective gene by, for example, homologous recombination, and usually contains a nucleotide sequence of a selectable marker gene for facilitating selection of a totipotent cell such as ES cell in which recombination has occurred. Examples of a positive selectable marker gene include a neomycin resistant gene, a β-galactosidase gene and the like. Examples of a negative selectable marker gene include a herpes simplex virus thymidine kinase gene, a diphtheria toxin A fragment gene and the like. Upon construction of the targeting vector, a commercially available plasmid vector for constructing a targeting vector may be utilized.

An example of a method for preparing a targeting vector which is preferably used in the present invention comprises using a selectable marker gene promoterless recombinant vector which carries a gene cassette containing a selectable marker gene from which a promoter region has been removed and which has a nucleotide sequence homologous to a part of a DNA of the PHF3 gene, replacing at least a part of a DNA within a protein-coding region of the PHF3 genome gene with a DNA which is a part or all of the vector and contains the gene cassette to prepare a selectable marker gene promoterless recombinant vector which carries a gene cassette containing a selectable marker gene from which a promoter region has been removed, and which has a nucleotide sequence homologous to a part of a DNA of the PHF3 gene on the both sides of the gene cassette. Another example of a method for preparing a targeting vector comprises using a selectable marker gene promoterless recombinant vector which carries a gene cassette containing a selectable marker gene from which a promoter region has been removed and which has a nucleotide sequence homologous to a part of a DNA of the PHF3 gene, inserting a DNA which is a part or all of the vector and contains the gene cassette into at least a part of a DNA within a protein-coding region of the PHF3 genome gene to prepare a selectable marker gene promoterless recombinant vector which carries a gene cassette containing a selectable marker gene from which a promoter region has been removed, and which has a nucleotide sequence homologous to a part of DNA of the PHF3 gene on the both sides of the gene cassette. In these cases, replacement or insertion may be performed in a plasmid vector for constructing a targeting vector into which the DNA to be replaced or inserted as described above has been incorporated in advance, or the DNA which has been already replaced or inserted as described above may be incorporated into a plasmid vector for constructing a targeting vector. When the PHF3 protein consists of the amino acid sequence of SEQ ID NO: 1, examples of the "at least a part of a DNA within a protein-coding region of the PHF3 genome gene" include nucleotides within the exon 3 region (herein, the "nucleotides within the exon 3 region" may be, for example, all nucleotides within the exon 3 region, or a part of nucleotides within the exon 3 region), and more preferably all nucleotides within the exon 3 region; provided that, in selection of exon herein, it is not preferable that the region of 2+3n (n is the number of amino acids within a region from the front position of exon 3) nucleotides from the front position of exon 3 is selected because in this case, a translation frame following exon 2 is consistent with a downstream gene of a protein-coding region of the PHF3 gene and thereby there is a possibility that a protein fused with the downstream region is produced.

Specifically, examples of the "gene cassette containing a selectable marker gene from which a promoter region has been removed" include a DNA (SA-IRES-βgeo-pH gene cassette) in which mouse EN2-derived SA (splicing accepter), encephalomyocarditis Virus-derived IRES (internal ribosome entry site), βgeo in which β-galactosidase and neomycin resistant genes are fused, and SV40-derived pA (polyadenylation signal) (Mountford P. et al., PNAS 91:4303-4307, 1994) are connected, and the like. A gene cassette containing a DNA comprising a nucleotide sequence encoding a selectable marker (i.e. a selectable marker gene from which a promoter region has been removed) is inserted, for example, between exon 2 and exon 3 of a genome gene encoding the PHF3 protein by homologous recombination utilizing the aforementioned targeting vector, so that it becomes impossible to synthesize a full length mRNA from the PHF3 gene and, consequently, the PHF3 gene can be disrupted. The gene cassette may contain a transcription termination signal (e.g. polyA addition signal) or the like, in addition to a DNA comprising a nucleotide sequence encoding a selectable marker. In addition, the gene cassette may have a part of a nucleotide sequence homologous to the DNA nucleotide sequence of the PHF3 genome gene in regions before and after a DNA comprising a nucleotide sequence encoding a selectable marker.

Herein, the "homologous nucleotide sequence" is not particularly limited as long as it is a nucleotide sequence which has identity or homology to a nucleotide sequence of a DNA to be compared and which does not cause a trouble upon implementation of a homologous recombination method. However, examples thereof include a nucleotide sequence which does not differ by continuous 3 or more nucleotides from a nucleotide sequence of a DNA to be compared and has 90% or more sequence identity to a nucleotide sequence of a DNA to be compared, preferably a nucleotide sequence which has 95% or more sequence identity to a nucleotide sequence of a DNA to be compared, more preferably a nucleotide sequence which has 100% sequence identity to a nucleotide sequence of a DNA to be compared.

For the aforementioned targeting vector, it is important to contain a gene cassette containing a selectable marker gene (e.g. a resistant gene to a cell toxin such as G418 etc., for example, a neomycin resistant gene) from which a promoter region has been removed and have a nucleotide sequence homologous to a part of the PHF3 gene, and the other factors are not particularly limited as long as they do not cause a trouble upon implementation of a homologous recombination method. In the present invention, the targeting vector is referred to as a selectable marker gene promoterless recombinant vector in some cases, as described above and below.

Then, the targeting vector is introduced into a totipotent cell such as an ES cell derived from a non-human animal such as a mouse according to a known method. Examples of such a gene introduction method include a known electric pulse method, liposome method, calcium phosphate method and the like. When the homologous recombination efficiency of an introduced gene is taken into consideration, an electric pulse method into a totipotent cell such as an ES cell is preferable.

The DNA of a totipotent cell such as an ES cell into which a gene has been introduced as described above is extracted, and the extracted DNA is subjected to Southern blotting analysis, PCR analysis and the like. Based on the results, a cell in which the aimed homologous recombination occurs between the wild-type genome gene of the PHF3 protein present on a chromosome (specifically, for example, at least a part of a DNA in a protein-coding region of the genome gene) and a DNA which is a part of the DNA of the targeting vector and contains the gene cassette and thereby a mutation is introduced into the wild-type genome gene of the PHF3 protein on a chromosome is selected. The selected ES cell and the wild-type ES cell are subjected to PCR real time quantification (RT-PCR method: Absolute Quantification Real-time Analysis) or the like, and the results can be compared to investigate an increase or a decrease in the expression amount of the PHF3 gene.

In the aforementioned method, after homologous recombination occurs in a chromosome in a totipotent cell such as an ES cell, only when a selectable marker is expressed in the ES cell utilizing a promoter of the PHF3 gene, the property of the selectable marker such as resistance function to a cell toxin is conferred on the cell. When the PHF3 gene is disrupted, the appearance efficiency of a clone in which homologous recombination occurs at the objective position to disrupt the PHF3 gene is enhanced by using said method, so that the objective clone can be effectively obtained.

The thus obtained totipotent cell such as an ES cell comprising the mutated genome gene is injected into the blastocyst of a wild-type female non-human animal such as a wild-type female mouse in the pseudo pregnant state according to a conventional microinjection method or aggregation method. Then, the resulting chimeric embryo is transplanted into the uterus of a foster parent. After embryonic differentiation is continued, for example, after 21 days in the case where the non-human animal is a mouse, a candidate chimeric non-human animal is born as an offspring. After the born candidate chimeric non-human animal is reared by a foster parent, a chimeric non-human animal (F0) comprising a mutated genome gene of the PHF3 protein introduced into a germ line cell is selected.

The selection may be performed by observing a difference in their fur color (the body hair color of the born chimeric non-human animals). (In this case, selection of a chimeric non-human animal comprising a high proportion of a recombinant totipotent cell such as a recombinant ES cell into which the mutated genome gene introduced becomes easy, so that the efficiency of acquiring a chimeric non-human animal comprising the mutated genome gene introduced into a germ line cell can be enhanced). Alternatively, the selection may be performed by extracting a DNA from a part of the body (e.g. the tip of a tail) and then subjecting the DNA to Southern blotting analysis, PCR analysis or the like.

A progeny (F1 obtained by mating a chimeric non-human animal comprising a mutated genome gene of the PHF3 protein introduced into a germ line cell with the wild-type non-human animal which belongs to the same animal species, is further subjected to Southern blotting analysis, PCR analysis or the like using a DNA extracted from a part of the body (e.g. the tip of a tail) as a material, and thereby, a heterozygote in which a mutated genome gene of the PHF3 protein is introduced (a non-human animal in which one of alleles of the diploid PHF3 gene is disrupted) is identified. In addition, an increase or a decrease in the expression amount of the PHF3 protein gene can be investigated, for example, by performing PCR real time quantification analysis or Northern blotting analysis using a RNA extract from a part of skin tissues such as a skin and a subcutaneous muscle, various fat tissues such as a peritesticular fat tissue, a retroperitoneal fat tissue, a mesenteric fat tissue, a subcutaneous fat tissue and a brown fat tissue, and other tissues such as heart, lung, kidney, gallbladder, liver, pancreas, spleen, intestine, orchis (testis), ovary, uterus, placenta, muscle, blood vessel, brain, marrow, thyroid gland, thymus, mammary gland and lymph node, or from an embryo at the embryonic stage as a material, and then comparing results. Further, increase and decrease in the amount of the PHF3 protein can be investigated by performing an ELISA (Enzyme Linked Immunosorbent Assay) method using the aforementioned body parts (mashed entity, section etc.) or body fluid (blood, urine etc.) as a material, and then comparing results.

The produced heterozygote comprising a mutated genome gene of the PHF3 protein stably possesses the mutated genome gene of the PHF3 protein in all of germ cells and somatic cells, and can effectively transmit the mutation to a progeny animal by mating or the like. Therefore, for progeny animals after F1, identification of heterozygotes, and investigation of increase and decrease in the expression amount of the gene and the amount of the protein which is the expression product of the gene can be performed in the same way as described above.

Generally, when a disrupted gene does not influence the development or differentiation of an embryonic individual and does not lead to embryonic death, in individuals after F1, genotypes of a homozygote (a non-human animal comprising the PHF3 gene whose all alleles in the diploid are disrupted or recombined), a heterozygote and the wild-type are produced at a probability in accordance with Mendel's laws. To the contrary, when a gene comprising a nucleotide sequence encoding the amino acid sequence of a protein essential for embryonic development or differentiation in the used non-human animal is disrupted, the survival and birth of a heterozygous or homozygous non-human animal are difficult, leading to embryonic death. This can be also inferred by comparing the actual birth rate and the predicted probability of appearance even when a heterozygote is obtained.

Generally, it is desirable that an animal used for experiment is an animal established as a line, that is, a genetically homogeneous animal. The genetic background of such homogeneous animal established as a line has been investigated in detail. In study using such experimental animals having the known genetic background, a treatment in the experimental animals produces a single effect, which can be easily identified. On the other hand, in a hybrid animal, various genes are present in the hetero state. Thus, a treatment in hybrid animals produces an effect as the sum of respective responses of individuals having respective genetic backgrounds to the treatment, which is extremely complicated. Therefore, it is difficult to determine whether an effect produced by a certain treatment is due to heterogeneity of the treatment or heterogeneity of the genetic background.

In the thus produced non-human animal or a progeny animal thereof (i.e. an individual of a knockout non-human animal or a progeny animal thereof), various blood biochemical tests, hemocyte tests and urine tests, various histopathological analyses, measurement of the total immunoglobulin E level in blood, and the like are performed, and thereby a difference in physiological action between the present knockout non-human animal and the wild-type non human animal (a non-human animal whose PHF3 gene is not disrupted) is investigated. Measurement of the total immunoglobulin E level in blood is a representative test which can be an index for assessment of atopic dermatitis.

As a result, as shown in Examples herein, in the present knockout non-human animal (e.g. 8 week-old mouse), formation of scabs and cell infiltration into the skin dermis layer are observed frequently as compared with the wild-type non-human animal. Further, the present knockout non-human animal (e.g. 43 week-old mouse) exhibits a finding of an extremely high level of the total immunoglobulin E in blood as compared with the wild-type non-human animal. From these findings, it can be confirmed that the present knockout non-human animal has the characteristic that exhibits abnormality in the immune system function and develops atopic dermatitis.

Based on the above results, the present knockout non-human animal or a part thereof and the wild-type non-human animal or a part thereof can be compared to know a difference in the development state of a disease with the development of atopic dermatitis.

Further, observation of growth and differentiation, development, and living behavior, a histopathological test or a biochemical test are performed on the present non-human animal or a part thereof during the period from the embryonic stage to death, and thereby the state of a disease associated with the PHF3 protein can be analyzed.

The present invention further provides a cultured animal cell comprising a disruption in at least one allele of the genome gene of the PHF3 protein comprising the amino acid sequence of SEQ ID NO: 7; more specifically, a primary cultured or established animal cell comprising a replacement of both or one of alleles of the diploid genome gene of a protein comprising the amino acid sequence of SEQ ID NO: 7 with a function-deficient mutant or a function-reduced mutant of the gene, or such a disruption (e.g. disruption by recombination) in all or one of alleles of the diploid genome gene as to delete or reduce its function, and a primary cultured or established animal cell comprising a replacement of all or one of alleles of the diploid genome gene of a protein comprising the amino acid sequence encoded by the nucleotide sequence of SEQ ID NO: 8 with a function-deficient mutant or a function-reduced mutant of the gene, or such a disruption (e.g. disruption by recombination) in all or one of alleles of the diploid genome gene as to delete or reduce its function.

The second aspect of the present invention relates to an animal model for a disease with the development of atopic dermatitis, comprising the present knockout non-human animal.

The present knockout non-human animal has a significantly reduced expression amount of the PHF3 protein as compared with the wild-type, and can express a disease associated with the protein. That is, as described above, the present knockout non-human animal specifically exhibits the following findings:
(a) formation of scabs on the skin
(b) cell infiltration into the skin dermis layer
(c) a high level of total immunoglobulin E in the blood
(d) variation in the expression amount of an atopic dermatitis-associated factor.

Therefore, the present knockout non-human animal can be used as an animal model for a disease with the development of atopic dermatitis, for 1) analysis of the state of a disease with the development of atopic dermatitis and the like, or 2) assessment of the atopic dermatitis treating effect or the atopic dermatitis development preventing effect of drugs, foods, drug candidates or food candidates.

Herein, "analysis of the state of a disease" means analysis of every change in a living body caused by a disease with the development of atopic dermatitis.

That is, the analysis of the state of a disease can be accomplished by performing observation of growth and differentiation, development, and living behavior, a histopathological test or a biochemical test during the period from the embryonic stage to death while comparing the present knockout non-human animal and the wild-type human animal. In the analysis of the disease state, the observation and tests may be performed according to a method usually used by a person skilled in the art, and "Transgenic Animal" edited by Kenichi Yamamura et al. (Kyoritsu Shuppan Co., Ltd.) and the like can be referenced.

For example, the total RNA extracted from a tissue of the present knockout non-human animal can be analyzed by PCR real time quantification analysis, Northern blotting analysis or the like, and then compared with analytical values of the wild-type non-human animal to identify a gene whose expression is induced or suppressed in the tissue. Based on the results, a marker gene whose expression is changed in a disease with the development of atopic dermatitis can be searched and, consequently, this can be used as an index for elucidating a causative factor of a disease.

Actually, as shown in Examples of the present invention, the present knockout non-human animal exhibits a change in the expression amount of an atopic dermatitis-associated factor. That is, the present knockout non-human animal exhibits an elevated expression of immunoglobulin E in blood, and thus the elevated expression of immunoglobulin E in blood can be used as an index to determine the presence or absence of a disease in a model animal for a disease with the development of atopic dermatitis.

A method for assessing an atopic dermatitis treating effect or an atopic dermatitis development preventing effect of drugs, foods, drug candidates or food candidates will be explained in detail below.

The third aspect of the present invention relates to a method for assessing an atopic dermatitis treating effect or the atopic dermatitis development preventing effect of a test substance using the present knockout non-human animal or a part thereof.

The produced non-human animal or a progeny animal or a part thereof (i.e. an individual of the knockout non-human animal, or a progeny animal thereof, or a part thereof) is useful in the field of development of drugs or foods for diseases associated with atopic dermatitis. In addition, the non-human animal or a progeny animal thereof, or a part thereof can be used for assessing atopic dermatitis controlling substances.

The assessment method of the present invention comprises:
(1) the first step of contacting the present non-human animal or a part thereof with a test substance,
(2) the second step of measuring an expression amount of an atopic dermatitis-associated factor in the non-human animal or a part thereof which has been contacted with the test substance, or an index value correlated with the expression amount, and then comparing the measured value with a control, and
(3) the third step of assessing an atopic dermatitis treating effect or an atopic dermatitis development preventing effect of the test substance on the basis of the comparison result of (2).

The atopic dermatitis controlling substance herein refers to a substance having an atopic dermatitis treating effect or an atopic dermatitis development preventing effect.

The "atopic dermatitis treating effect or atopic dermatitis development preventing effect" means a treating effect or a development preventing effect on atopic dermatitis based on activity of ameliorating symptoms characteristic of atopic dermatitis, for example, formation of scabs, cell infiltration into the skin dermis layer and a high level of total immunoglobulin E in blood.

Herein, the test substance is not particularly limited, and may be a nucleic acid, a peptide, a protein (including an antibody directed to the present protein), an organic compound, an inorganic compound or the like. Examples of the test substance include a cell extract, an expression product of a gene library, a synthetic low-molecular organic compound, a synthetic peptide, a synthetic nucleic acid, a natural compound and the like. Herein, examples of the "antibody directed to the present protein" include antibodies immunologically specific for a protein comprising the amino acid sequence of SEQ ID NO: 1 or 7, or a partial region thereof.

Herein, the "contacting the present knockout non-human animal or a part thereof with a test substance" means administrating a test substance to the non-human animal, or contacting a test substance with a part of the non-human animal, and can be performed by a method generally used by a person skilled in the art.

When the test substance is administered to the non-human animal, a method for administering is not particularly limited, and may be oral or parenteral administration. Examples of a parenteral administration method include intravenous administration, subcutaneous administration, intracutaneous administration, intraperitoneal administration (ip), rectal administration, transdermal administration (application) and the like.

The form of the test substance is not particularly limited, and can be used as a solid, a liquid, a mixture with a base, a suspension, a solution or the like. When the test substance is formulated into a suspension or a solution, water, a pH buffer, a methylcellulose solution, a physiological saline, an organic solvent aqueous solution (as an organic solvent, ethanol and dimethyl sulfoxide are usually used) or the like is used. Examples of the base include oils such as glycerin, squalane and the like, and these are mainly used for preparing a test substance for application.

Examples of a method for measuring the "expression amount of an atopic dermatitis-associated factor" include a method comprising measurement of the RNA amount of the factor and a method comprising measurement of the protein amount of the factor, and said method may be performed in the same manner as a method for measuring the expression amount of an atopic dermatitis-associated factor as described below or a method described in Examples.

Examples of the "index value correlated with the expression amount of an atopic dermatitis-associated factor" in the second step of the assessment method include:
(a) formation of scabs on the skin,
(b) cell infiltration into the skin dermis layer, and
(c) the total immunoglobulin E level in blood.
   For the heterozygote of the present invention, an additional example of the index value is the expression amount of PHF3.

An example of a method for measuring formation of scabs on the skin comprises observation with naked eyes.

An example of a method for measuring cell infiltration into the skin dermis layer comprises fixing a section of the skin or bone marrow, HE-staining the section to prepare a specimen, and then observing the specimen.

As a method for measuring an atopic dermatitis-associated factor, a method using an ELISA kit well-known to a person skilled in the art, or the like can be used.

Besides, examples of a procedure for biochemically analyzing the present knockout non-human animal include a method well-known to a person skilled in the art, and a method described in "The Journal of Clinical Investigation, April 2002, vol.109, no.8 (authored by Robert V. Farese Jr.et al.)" or "Nature Genetics, vol.25, May 2000 (authored by Robert V. Farese Jr. et al.)".

Herein, the "atopic dermatitis-associated factor" is, for example, a factor involved in the activation process of a T cell by antigen stimulation, a factor involved in the activation process of a B cell by a T cell, a factor involved in inflammation, or the like, and examples include one or more factors selected from IFN-γ, IL-4, IL-5, IL-13, IL-18, Immunoglobulin E and the like. When the expression amount of the atopic dermatitis-associated factor is measured, RNA or a protein may be used as a subject to be measured. It is preferable that a test substance which reduces the amount of IFN-γ, IL-4, IL-5, IL-13, IL-18 or immunoglobulin E is selected as a candidate substance of a remedy for an allergy disease or a prophylactic for the development of an allergy disease.

Each of them will be described in detail below.
1) The case where RNA is used as a subject to be measured
   In the case where an RNA is utilized as a biological sample, in order to measure the expression amount of the atopic dermatitis-associated factor, the expression level of an IFN-γ gene, an IL-4 gene, an IL-5 gene, an IL-13 gene or an IL-18 gene per unit amount of the total RNA contained in the biological sample may be detected and then measured.
   The animal species from which the atopic dermatitis-associated factor gene is derived is not particularly limited. Usually, the gene derived from the same species as the present non-human animal is used.
   Examples of the IFN-γ gene include a mouse-derived IFN-γ gene (Genbank Accession No. NM_008337), a human-derived IFN-γ gene (Genbank Accession No. NM_000619) and the like.
   Examples of the IL-4 gene include a mouse-derived IL-4 gene (Genbank Accession No. NM_021283), a human-derived IL-4 gene (Genbank Accession No. NM_000589) and the like.
   Examples of the IL-5 gene include a mouse-derived IL-5 gene (Genbank Accession No. NM_010558), a human-derived IL-5 gene (Genbank Accession No. NM_000879) and the like.
   Examples of the IL-13 gene include a mouse-derived IL-13 gene (Genbank Accession No. NM_008355), a human-derived IL-13 gene a human-derived IL-13 gene (Genbank Accession No. NM_002188) and the like.
   Examples of the IL-18 gene include a mouse-derived IL-18 gene (Genbank Accession No. NM_008360), a human-derived IL-18 gene (Genbank Accession No. NM_001562) and the like.
   In the present non-human animal, a progeny thereof or a part thereof, the presence or absence or the extent of expression of the atopic dermatitis-associated factor gene (or the gene product) can be measured by detecting the presence or absence or the extent of expression of the gene.
   An increase or a decrease in the expression amount of the atopic dermatitis-associated factor gene or a trend in the expression amount of other genes associated therewith is investigated by performing PCR real time quantification analysis or Northern blotting analysis using an RNA extracted from each tissue of the present knockout non-human animal as a sample, and thereby the development state of a disease with the development of atopic dermatitis can be known. Consequently this investigation can be used as an index for elucidating a causative factor of the disease.
   Specifically, a biological sample derived from the present non-human animal is contacted with a primer or probe derived from an atopic dermatitis-associated factor gene, and the amount of an RNA binding with the primer or probe can be measured by a known method such as a Northern blotting method, a RT-PGR method, a DNA chip analysis, an in situ hybridization analysis or the like. Examples of the primer or probe include a polynucleotide comprising at least 15 continuous nucleotides in the nucleotide sequence of the atopic dermatitis-associated factor gene, and/or a polynucleotide complementary to them.
   Examples of a polynucleotide used as the primer include polynucleotides having a nucleotide length of usually 15 bp to 100 bp, preferably 15 bp to 50 bp, more preferably 15 bp to 35 bp. Examples of a polynucleotide used as the detection probe include polynucleotides having a nucleotide length of usually 15 bp to the full length, preferably 15 bp to 1 kb, more preferably 100 bp to 1 kb.
   When a Northern blotting method is utilized, specifically, for example, the probe is labeled with a radioactive isotope element (32P, 33P etc.: RI) or a fluorescent substance, and the labeled probe is hybridized with an RNA derived from a living body tissue of a subject which has been transferred to a nylon membrane or the like according to a conventional method. A duplex formed between the primer (DNA or RNA) derived from an atopic dermatitis-associated factor and the total RNA derived from a living body sample is measured by detecting a signal derived from the label of the primer (RI or fluorescent substance) with a radiation detector (BAS-1800II, manufactured by Fuji Photo Film Co., Ltd.) or a fluorescent light detector. Alternatively, a measuring method which comprises labeling a probe DNA, hybridizing the probe with RNA derived from a biological sample and then detecting a signal derived from the label of the probe with a multibioimager STORM860 (manufactured by Amersham Pharmacia Biotech) using AlkPhos Direct Labelling and Detection System (manufactured by Amersham Pharmacia Biotech) according to the protocol may be used.
   When a RT-PCR method is utilized, a method which comprises hybridizing the primer with an RNA derived from a biological sample, performing a PCR method according to a conventional method and then detecting an amplified double-stranded DNA can be exemplified. For detecting the amplified double-stranded DNA, a method which comprises detecting a produced labeled double-stranded DNA by performing the PCR using a primer which has been labeled with RI or a fluorescent substance in advance, a detecting method which comprises transferring a produced double-stranded DNA to a nylon membrane or the like according to a conventional method, and hybridizing a labeled disease marker as a probe with the nylon membrane or the like, or the like can be used. The produced labeled double-stranded DNA product can be measured with an Agilent 2100 bioanalyzer (manufactured by Yokokawa Analytical systems) or the like. Alternatively, a RT-PCR reaction solution is prepared with SYBR Green RT-PCR Regents (manufactured by Applied Biosystems) according to the protocol, and reacted with ABI PRIME 7700 Sequence Detection System (manufactured by Applied Biosystems), thereby, the reaction product may be detected.
   When DNA chip analysis is utilized, a method which comprises preparing a DNA chip on which the primer or probe is stuck as a DNA probe (single-stranded or double-stranded), hybridizing the chip with a cRNA prepared from an RNA derived from a biological tissue according to a conventional method, and then binding a duplex formed between the DNA and the cRNA to a labeled probe prepared from the primer or probe to detect the RNA can be exemplified. As the DNA chip, a DNA chip which can detect or measure the gene expression level of an IFN-γ gene, an IL-4 gene, an IL-5 gene, an IL-13 gene, an IL-18 gene or an immunoglobulin E gene may be used.
2) The case where a protein is used as a subject to be measured
   In the case where a protein is utilized as a biological sample, in order to measure the expression amount of the atopic dermatitis-associated factor, the expression level of IFN-γ, IL-4, IL-5, IL-13, IL-18 or immunoglobulin E per unit amount of the total protein contained in the biological sample may be detected and then measured.
   The animal species from which the atopic dermatitis-associated factor (protein) is derived is not particularly limited. Usually, the protein derived from the same species as the present non-human animal is used.
   Examples of the IFN-γ include mouse-derived IFN-γ (Genbank Accession No. NP_0032363), human-derived IFN-γ (Genbank Accession No. NP_000610) and the like.
   Examples of the IL-4 include mouse-derived IL-4 (Genbank Accession No. NP_067258), human-derived IL-4 (Genbank Accession No. NP_000580) and the like.
   Examples of the IL-5 include mouse-derived IL-5 (Genbank Accession No. NP_034688), human-derived IL-5 (Genbank Accession No. NP_000870) and the like.
   Examples of the IL-13 include mouse-derived IL-13 (Genbank Accession No. NP_032381), human-derived IL-13 (Genbank Accession No. NP_002179) and the like.
   Examples of the IL-18 include mouse-derived IL-18 (Genbank Accession No. NP_032386), human-derived IL-18 (Genbank Accession No. NP_001553) and the like.
   Examples of immunoglobulin E include immunoglobulin E derived from human (Genbank Accession No. XP_370382) and the like.

In the case where a solution containing a protein is utilized as a biological sample, for example, an atopic dermatitis-associated factor contained in the biological sample is reacted with an antibody capable of recognizing the atopic dermatitis-associated factor, and the amount of the atopic dermatitis-associated factor capable of binding to the antibody is detected and then measured.

The animal species from which an antibody capable of recognizing an atopic dermatitis-associated factor is derived is not particularly limited. Usually, an antibody obtained by using an atopic dermatitis associated factor derived from the same species as the present non-human animal as an antigen is used.

The antibody of the atopic dermatitis-associated factor is not particularly limited in its form, but may be a polyclonal antibody or a monoclonal antibody directed to the atopic dermatitis-associated factor as an immunological antigen. Further, an antibody having the antigen binding property to a polypeptide comprising continuous at least about 3 amino acids, usually about 8 amino acids, preferably about 15 amino acids of the amino acid sequence constituting the atopic dermatitis-associated factor may be used.

These antibodies can be produced according to a conventional method (Current protocols in Molecular Biology edit. Ausubel et al. (1987) Publish. John Wiley and Sons. Section 11.12 - 11.13). Specifically, in the case of a polyclonal antibody, a non-human animal such as a domestic rabbit is immunized using IFN-γ, IL-4, IL-5, IL-13, IL-18 or immunoglobulin E which has been expressed in Escherichia coli or the like and purified according to a conventional method, or using an oligopeptide comprising a partial amino acid sequence of any of the proteins synthesized according to a conventional method. An objective polyclonal antibody can be obtained from the serum of the immunized animal according to a conventional method. On the other hand, in the case of a monoclonal antibody, a non-human animal such as a mouse is immunized with IFN-γ, IL-4, IL-5, IL-13, IL-18 or immunoglobulin E which has been expressed in Escherichia coli or the like and purified according to a conventional method, or an oligopeptide comprising a partial amino acid sequence of these proteins. The spleen cells obtained from the immunized animal are fused to myeloma cells to prepare hybridoma cells, and then, an objective monoclonal antibody can be obtained from among the hybridoma cells (Current protocols in Molecular Biology edit. Ausubel et al. (1987) Publish. John Wiley and Sons. Section 11.4 - 11.11).

A protein used in production of an antibody can be obtained by performing DNA cloning based on sequence information on a gene of the atopic dermatitis-associated factor, and then performing manipulations for construction of an expression plasmid, transfection into a host, culture of a transformant, and collection of a protein from the culture. These manipulations can be performed according to a method known to a person skilled in the art, or a method described in literatures (Molecular Cloning, T. Maniatis et al., CSH Laboratory (1983), DNA Cloning, DM. Glover, IRL PRESS (1985)). Specifically, for example, a recombinant DNA (expression vector) capable of expressing a gene encoding the atopic dermatitis-associated factor in a desired host cell is made and then introduced into a host cell to transform it. The transformant is cultured, and the objective protein is collected from the resulting culture. The atopic dermatitis-associated factor can be also produced based on information of the amino acid sequence thereof by a general chemical synthesis method (peptide synthesis). Specifically, a solution phase synthesis method and a solid phase synthesis method described in "Basis and Experiment of Peptide Synthesis" (Nobuo Izumiya et al., Maruzen, Published in 1987) can be used.

The atopic dermatitis-associated factor utilized in the present invention includes not only a protein comprising the aforementioned amino acid sequence, but also homologues thereof. Examples of the homologue include proteins comprising the aforementioned amino acid sequence of the protein in which one or more amino acids are deleted, substituted or added, and having the biological function equivalent to the function of the protein, and/or having the immunological activity equivalent to the protein comprising the aforementioned amino acid sequence. Herein, "the immunological activity equivalent" includes a protein having the ability to bind specifically to an antibody directed to the atopic dermatitis-associated factor.

The "control" in the second step of the present assessing method means, for example,
(a) the case where a wild-type non-human animal which belongs to the same animal species as the present non-human animal used in the assessment method is subjected to the same steps as the first step and the second step, or
(b) the case where a control substance (positive control, negative control) in place of a test substance is used in the same steps as the first step and the second step.

In the case of the (a), when the atopic dermatitis treating effect or the atopic dermatitis development preventing effect in the present non-human animal is equivalent to or more than the atopic dermatitis treating effect or the atopic dermatitis development preventing effect of a test substance in the wild-type non-human animal, the test substance can be assessed to have an atopic dermatitis treating effect or an atopic dermatitis development preventing effect. When the atopic dermatitis treating effect or the atopic dermatitis development preventing effect in the present non-human animal is less than the atopic dermatitis treating effect or the atopic dermatitis development preventing effect of a test substance in the wild-type non-human animal, the test substance can be assessed not to have an atopic dermatitis treating effect or an atopic dermatitis development preventing effect due to the PHF3 protein.

In the case of the (b), examples of the control substance include a positive control and a negative control. The positive control refers to an arbitrary substance having an atopic dermatitis treating effect or an atopic dermatitis development preventing effect and, specific example thereof is corticosteroid or the like. Examples of the negative control include a solvent contained in a test substance, a test system solution which is a background, and the like.

In the case of the negative control as the control substance, when the atopic dermatitis treating effect or the atopic dermatitis development preventing effect of a test substance is more than the atopic dermatitis treating effect or the atopic dermatitis development preventing effect of the control substance, the test substance can be assessed to have an atopic dermatitis treating effect or an atopic dermatitis development preventing effect. On the other hand, when the atopic dermatitis treating effect or the atopic dermatitis development preventing effect of a test substance is equivalent to or less than the atopic dermatitis treating effect or the atopic dermatitis development preventing effect of the control substance, the test substance can be assessed not to have an atopic dermatitis treating effect or an atopic dermatitis development preventing effect.

In the case of the positive control as the control substance, the extent of the atopic dermatitis treating effect or the atopic dermatitis development preventing effect of a test substance can be assessed by comparing the atopic dermatitis treating effect or the atopic dermatitis development preventing effect of a test substance with the atopic dermatitis treating effect or the atopic dermatitis development preventing effect of the control substance.

Since substances (atopic dermatitis controlling substances) selected based on the atopic dermatitis treating effect or the atopic dermatitis development preventing effect of test substances determined by the present assessing method have an atopic dermatitis treating effect or an atopic dermatitis development preventing effect, they can be used as drugs or foods effective for treatment or prevention of a diseases with the development of atopic dermatitis or the like. Further, compounds derived from substances obtained by the present screening method can be also used similarly.

Substances obtained by the present screening method may form salts. Examples of the salt of the substance include salts with physiologically acceptable acids (e.g. inorganic acids, organic acids) or bases (e.g. alkali metal), and inter alia, physiologically acceptable acid addition salts are preferred. Examples of such salt include salts with inorganic acids (e.g. hydrochloric acid, phosphoric acid, hydrobromic acid, sulfuric acid), and salts with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid). Substances obtained by the present screening acid can be used orally as optionally sugar-coated tablets, capsules, elixirs, microcapsules or the like, or parenterally as the form of injectables such as sterile solutions or suspensions with water or other pharmaceutically acceptable liquid. These preparations can be prepared by mixing the substance with a physiologically acceptable carrier, flavor, excipient, vehicle, antiseptic, stabilizer, binder and the like in a unit dosage form required in generally recognized pharmaceutical practice. The amount of an active ingredient in these preparations is such an amount that a suitable volume in the indicated range is obtained. Examples of an additive which can be incorporated into a tablet, a capsule or the like include binders such as gelatin, corn starch, tragacanth, and gum arabic; excipients such as crystalline cellulose; swelling agents such as corn starch, gelatin, alginic acid and the like; lubricants such as magnesium stearate; sweeteners such as sugar, lactose and saccharine; flavors such as peppermint, Gaultheria adenothrix oil (akamono oil) and cherry, and the like. When the dosage unit form is a capsule, it may contains a liquid carrier such as a fat or oil in addition to the aforementioned type of materials. A sterile composition for injection can be formulated according to conventional pharmaceutical practice, for example by dissolving or suspending an active substance in a vehicle such as water for injection, and a naturally occurring vegetable oil such as a sesame oil, a palm oil or the like. As an aqueous liquid for injection, a physiological saline, an isotonic solution containing glucose or other auxiliary agent (e.g. D-sorbitol, D-mannitol etc.) is used, and may be used in combination with an alcohol (e.g. ethanol), a polyalcohol (e.g. propylene glycol, polyethylene glycol), a nonionic surfactant (e.g. Polysorbate 80 TM, HCO-50) or the like. As an oily liquid, for example, a sesame oil, a soybean oil or the like is used, and may be used in combination with benzyl benzoate, benzyl alcohol or the like which is a solubilizer. In addition, the aforementioned remedies or development prophylactics may contain, for example, a buffer (e.g. phosphate buffer, sodium acetate buffer), a soothing agent (e.g. benzalkonium chloride, procaine hydrochloride etc.), a stabilizer (e.g. human serum albumin, polyethylene glycol etc.), a preservative (e.g. benzyl alcohol, phenol etc.), an antioxidant and the like. The prepared pharmaceutical composition such as an injectable is usually filled into a suitable ample.

Since the thus obtained preparations are safe and low toxic, they can be administered to a human or a mammal (e.g. rat, rabbit, sheep, pig, cow, cat, dog, monkey etc.). A dose of the substance varies depending on a disease to be treated, a subject of administration, a route of administration and the like. For example, when the compound is orally administered, generally, the substance is administered in an amount of about 0.1 to 100 mg, preferably about 1 to 50 mg, more preferably about 1 to 20 mg per day to an adult (weight 60 kg). When parenterally administered, a dose per time of the substance varies depending on a subject of administration, a disease to be treated and the like. For example, when the substance is usually administered to an adult (weight 60 kg) in the form of an injctable, the substance is conveniently administered in an amount of about 0.01 to 30 mg, preferably about 0.1 to 20 mg, more preferably about 0.1 to 10 mg per day via intravenous injection. In the case of other non-human animals, an equivalent amount calculated on the basis of the aforementioned dose per 60 kg body weight can be administered.

The present invention further provides use of:
(1) the PHF3 protein or a partial region thereof;
(2) a polynucleotide comprising a nucleotide sequence encoding the amino acid sequence of the protein or a partial region thereof described in (1), or a nucleotide sequence complementary to the nucleotide sequence; and
(3) a DNA encoding a RNA having a ribozyme activity which cleaves specifically a nucleotide sequence encoding the amino acid sequence of the protein or a partial region thereof described in (1); and
(4) a DNA encoding a RNA which suppresses the expression of the PHF3 gene on the basis of RNAi effect when expressed in a cell, for protein therapy or gene therapy of an allergy disease.

Herein, preferable examples of the PHF3 protein can include proteins which have an activity for ameliorating allergy disease symptoms and comprise any one of the following amino acid sequences:
<Amino acid sequence>
(a) the amino acid sequence of SEQ ID NO: 1 or 7;
(b) the amino acid sequence of SEQ ID NO: 1 or 7 in which one or more amino acids are deleted, added or substituted;
(c) an amino acid sequence with 75% or more sequence identity to the amino acid sequence of SEQ ID NO: 1 or 7;
(d) the amino acid sequence encoded by the nucleotide sequence of SEQ ID NO: 2 or 8;
(e) an amino acid sequence encoded by a DNA comprising a nucleotide sequence with 80% or more sequence identity to the nucleotide sequence of SEQ ID NO: 2 or 8; and
(f) an amino acid sequence encoded by a DNA which hybridizes with a DNA comprising a nucleotide sequence complementary to the nucleotide sequence of SEQ ID NO: 2 or 8 under stringent conditions, and the like.

As shown in Examples described hereinbelow, since the PHF3 gene is highly expressed in human leukocytes and the expression is reduced by stimulation with a mitogen, the activation state of a leukocyte and the PHF3 gene expression have a close relationship. Therefore, introduction of the protein, the polynucleotide or the like into a body results in a compensated expression amount of the PHF3 gene, so that improvement in the symptoms of an allergy disease is expected.

When the PHF3 protein or the like is used as a drug, a protein whose antigenecity to a human is the lowest among the PHF3 protein and the like and which has the amino acid sequence of the PHF3 protein or at least a part thereof is preferably used as an active ingredient of a pharmaceutical composition.

The PHF3 protein can be prepared as a natural protein from a naturally-occurring organism by manipulation such as extraction and purification, or can be prepared as a recombinant protein using a genetic engineering procedure. For example, a crude extract can be prepared from a human cell or tissue, and then purified using a variety of columns to prepare the purified protein. Herein, the cell is not particularly limited as long as it is producing the PHF3 protein and, for example, a leukocyte-derived cell or the like can be used.

Further, based on a nucleotide sequence of the PHF3 gene, an antisense drug can be developed. That is, a part of the PHF3 gene or a derivative thereof synthesized by a known method can used to regulate the expression of the PHF3 protein. An oligonucleotide comprising a sequence complementary to the PHF3 gene or a derivative thereof can be also used to regulate the expression of the PHF3 protein.

The present invention further provides use of:
(1) the PHF3 protein or a partial region thereof;
(2) a polynucleotide comprising a nucleotide sequence encoding the amino acid sequence of the protein or a partial region thereof described in (1), or a nucleotide sequence complementary to the nucleotide sequence; and
(3) a gene of the PHF3 protein, for diagnosing atopic dermatitis.

Herein, preferable examples of the PHF3 protein include proteins which have an activity for ameliorating allergy disease symptoms and comprise any one of the following amino acid sequences:
<Amino acid sequence>
(a) the amino acid sequence of SEQ ID NO: 1 or 7;
(b) the amino acid sequence of SEQ ID NO: 1 or 7 in which one or more amino acids are deleted, added or substituted;
(c) an amino acid sequence with 75% or more sequence identity to the amino acid sequence of SEQ ID NO: 1 or 7;
(d) the amino acid sequence encoded by the nucleotide sequence of SEQ ID NO: 2 or 8;
(e) an amino acid sequence encoded by a DNA comprising a nucleotide sequence with 80% or more sequence identity to the nucleotide sequence of SEQ ID NO: 2 or 8; and
(f) an amino acid sequence encoded by a DNA which hybridizes with a DNA comprising a nucleotide sequence complementary to the nucleotide sequence of SEQ ID NO: 2 or 8 under stringent conditions.

Herein, the gene of the PHF3 protein refer to those having a broad meaning, including in addition to a nucleotide sequence encoding the protein described above in (1), a transcription regulating region present upstream of the nucleotide sequence.

As described above, since the PHF3 gene is highly expressed in human leukocytes and the expression is reduced by stimulation with a mitogen, the activation state of a leukocyte and the PHF3 gene expression have a close relationship. Therefore, the expression amount of the PHF3 gene can be an index for diagnosis of atopic dermatitis. The aforementioned diagnosis of atopic dermatitis includes, for example, so-called genetic diagnosis (e.g. including mRNA level diagnosis, genome level diagnosis etc.) for detecting susceptibility, and provides use relating to technique including also a method of detecting a genetic mutation or gene polymorphism in the gene of the PHF3 protein or a transcription regulating region of the gene, as a means for diagnosing sensitivity of an allergy disease.

Specific examples of such method include a hybridization method using a probe of a DNA which has a nucleotide sequence of the gene of the PHF3 protein and is labeled with an enzyme such as horseradish peroxidase (HRP) or the like, a radioactive isotope element, a fluorescent substance, a chemiluminescent substance or the like, and a PCR method using primers which is designed based on a nucleotide sequence of the DNA.

Herein, the physiological activity relating to a transcription regulating region of the gene of the PHF3 protein may be assessed, for example, by a reporter gene assay method which comprises making a recombinant DNA molecule in which a suitable reporter gene is connected to the downstream of a DNA comprising the nucleotide sequence of the transcription regulating region, transforming a suitable host cell with the recombinant DNA molecule, and then detecting and quantitating a reporter gene product produced from the resulting transformant, a known gel shift assay method using a labeled DNA comprising the nucleotide sequence of the transcription regulating region, or the like. Such assay method can be also applied for screening of substances which inhibit or activate the expression of the PHF3 gene.

### Examples

The present invention will be explained below by Examples, but the present invention is not limited to them.

### Example 1 (Preparation of PHF3 gene knockout ES cell)

PHF3 gene knockout ES cells were prepared by a gene trap method (International Publication WO01/005987) using a trap vector pU-17.

It was confirmed that a disrupted or recombined gene is only the PHF3 gene, according to the following method.

In the ES cell used, it was confirmed by PCR analysis that only a copy of the vector was introduced, and it was also confirmed by Southern blotting analysis that the full length of the vector was introduced. Further, when a partial sequence of the trapped gene (a cDNA sequence upstream of an insertion position of the trap vector) was determined by a 5' RACE method, it was consistent with the nucleotide sequences of the first exon and the second exon of the PHF3 gene (GenBank Acc. No. XM_129836) (see Fig. 1). The mouse PHF3 gene is composed of 16 exons, and encodes a protein consisting of 2025 amino acids. A translation initiation codon of the gene is present in the second exon. In a cell in which the trap vector pU-17 is inserted immediately after the second exon, a protein in which the PHF3 protein is fused with βgeo immediately after the start of translation is translated, and the original PHF3 protein is not made. Then, the nucleotide sequence of the genome gene of the ES cell used was analyzed. As a result, it was confirmed that the trap vector pU-17 was inserted between the second exon and the third exon. Thereby, it was found that the ES cell was a cell deficient in the expression of the PHF3 gene due to alteration of the gene on a chromosome.

### Example 2 (Production of PHF3 gene knockout mouse)

A chimeric mouse comprising cells in which the PHF3 gene was knocked out was produced according to the following method. The ES cell prepared in Example 1 in which the PHF3 gene was disrupted or recombined and an ICR line 8-cell stage embryo were aggregated by an aggregation method (Nagy A., 1993, Oxford University Press, Oxford, pp147-179). On the following day, the chimeric embryo which had been developed into a morula or a blastocyst was transplanted into the uterus of a pseudo-pregnancy-induced ICR line female mouse (pseudo-pregnancy induction day 3) (Shinichi Aizawa: Gene targeting: Preparation of Mutated Mouse using ES Cell, Yodosha Co., Ltd, 1995). After 17.5 days from transplantation, the female mouse was subjected to caesarotomy to take out a fetal, and thereby a chimeric mouse was obtained.

The chimeric mouse was reared by a foster parent for 4 weeks (Shinichi Aizawa: Gene targeting: Preparation of Mutated Mouse using ES Cell, Yodosha Co., Ltd, 1995). The chimeric mouse was weaned at 4 week, and mated with two ICR line female mice at 6 week to confirm germline transmission (GT). A tissue of a baby born between the chimeric mouse and ICR was taken. The genome DNA of the tissue was subjected to PCR analysis to confirm the presence or absence of the introduced gene, and subjected to Southern analysis to confirm whether the gene of the ES cell and the gene of the mouse are consistent or not. The chimeric mouse for which GT determination had been completed was mated naturally with two C57BL/6J female mice to produce F1 heterozygotes of the PHF3 gene knockout mouse.

Fertilized eggs obtained by in vitro fertilization between the produced F1 heterozygous male knockout mouse and a hyperovulation-treated C57BL/6J female mouse were transplanted into a pseudo-pregnancy-induced ICR line female mouse (recipient) to produce a large number of F2 heterozygous knockout mice. IVF was performed using the produced F2 heterozygous knockout mouse to produce fertilized eggs. The embryo was transplanted into a recipient to produce a F3 homozygous knockout mouse.

The appearance of the thus produced PHF3 gene homo-knockout mouse (33 week-old) was observed. An example is shown in Fig. 2. In the mouse, hair loss, formation of scabs over a wide range of the back part and itching behavior were observed, which means atopic dermatitis symptoms were recognized.

### Example 3 (Pathological test of PHF3 gene homo-knockout mouse)

The atopic dermatitis symptoms found in the PHF3 gene homo-knockout mouse produced in Example 2 were histopathologically tested according to the following method. After the 8 week-old PHF3 gene homo-knockout mouse was led to death by exsanguination under ether anesthesia, a part of a skin tissue (abdominal) was taken from the mouse and fixed in a 10% neutral buffered formalin solution at room temperature for 1 week. The fixed skin tissue was alcohol-dehydrated, then embedded in paraffin, and thinly sliced at a thickness of 2 to 10 µm using a microslicer. The resulting thin section was placed on a slide glass. The thin section was de-paraffined (7 min x 3) with xylene, washed with 100% ethanol (30 sec x 1, 2 min x 2) and then distilled water, and then stained with hematoxylin/eosin (hereinafter, referred to as HE staining). The HE staining was performed by immersing the section in a hematoxylin solution (a solution of hematoxylin 2 g, aluminum potassium sulfate 75 g, sodium iodate 0.4 g, citric acid 1 g and chloral hydrate 50 g in 1L of distilled water) for 10 minutes, washing the section with distilled water for 15 minutes, immersing the section in an eosin solution (a mixture of a 1% eosin aqueous solution 200 mL, a 1% phloxine B aqueous solution 5 mL and 80% ethanol 615 mL, which was adjusted to pH 5.5 with acetic acid) for 2 minutes, rinsing it with distilled water again for 15 second, and then dehydrating it sequentially with 80% ethanol (15 seconds), 90% ethanol (15 seconds), 100% ethanol (2 min x 2) and xylene (5 min x 3). Thereafter, the HE-stained skin tissue was subjected to a histopathological test using a light microscope. Results are shown in Fig. 3. In the PHF3 gene homo-knockout mouse, proliferation of prickle cells, formation of scabs, and cell infiltration into the skin dermis layer were observed and, additionally, cell infiltration into a subcutaneous tissue and atrophy of cutaneous muscle were observed. Thereby, it was confirmed that the PHF3 gene homo-knockout mouse exhibits atopic dermatitis symptoms.

### Example 4 (Measurement of immunoglobulin E level)

The total immunoglobulin E (IgE) level in the serum of the PHF3 gene homo-knockout mouse was studied. About 40 µl of blood was taken from the tail vein of the mouse, and the serum was separated by a conventional method. The IgE level in the serum was measured with Lewis IgE-ELISA KIT (mouse) manufactured by Shibayagi Co., Ltd according to the method recommended by the kit. Results are shown in Fig. 4. The 43 week-old PHF3 gene homo-knockout mouse individual had a high level of total IgE in serum. To the contrary, the wild-type mouse had a low level of total IgE in serum. Therefore, it was found that the dermatitis caused by disruption or recombination of the PHF3 gene produces an increased level of IgE, which is a symptom peculiar to atopic dermatitis.

### Example 5 (Analysis of PHF3 gene expression in leukocytes (in vitro))

The presence or absence of PHF3 mRNA in an RNA derived from a human leukocyte specimen was studied by a reverse transcription-PCR method. PCR was performed for 32 cycles in a 25 µl reaction system using each 12.5 pmol of a primer set for specific amplification of the PHF3 protein, 1 µl of a human normal leukocyte (mononuclear cell, killer T cell, helper T cell, monocyte, B cell)-derived cDNA (manufactured by Clontech) as a template, and ExTaq DNA polymerase (manufactured by TAKARA SHUZO Co., Ltd.). Of the leukocyte samples, the samples marked with "resting" are cDNAs derived from hemocytes which were subjected to only a treatment of separation from blood, and the samples marked with "activated" are cDNAs derived from a mononuclear cell treated with concanavalin A (ConA) and pokeweed mitogen (PWM), a helper T cell treated with ConA, a killer T cell treated with phytohemagglutinin, or a B cell treated with PWM. In the PCR, hPHF3 F1 (SEQ ID NO: 3: gtggcaatga taccattgat ga) was used as a sense primer, and hPHF3 R2 (SEQ ID NO: 4: cacttcatct acacaactag gca) was used as an antisense primer. An amplified DNA from the PHF3 mRNA is a DNA having 821 base pairs. The amount of a β actin mRNA was also measured using the same template cDNA. PCR was performed for 25 cycles using β actin F1 (SEQ ID NO;5: accaactggg acgacatgga gaa) as a sense primer and β actin R1 (SEQ ID NO: 6: gtggtggtga agctgtagcc) as an antisens primer. An amplified DNA from a β actin mRNA is a DNA having 380 base pairs. Results are shown in Fig. 5.

The PHF3 mRNA was highly expressed in untreated (resting) leukocytes, while the expression amount was remarkably reduced in a helper T cell treated with concanavalin A, a killer T cell treated with phytohemagglutinin and B cell treated with pokeweed mitogen. The expression amount of β actin mRNA was approximately the same extent in each sample. From these results, it was found that the expression of PHF3 gene is suppressed in activated leukocyte cells.

### Example 6 (Analysis of expression of PHF3 gene in knockout mouse)

It was studied by a PCR method that transcription of the PHF3 gene is suppressed in the PHF3 gene homo-knockout mouse produced by the present invention.

The submandibular lymph nodes of three PHF 3 gene homo-knockout mice (#3004-3006) and wild-type mice (#3024-3026) were removed. The total RNAs were prepared from the submandibular lymph nodes using RNeasy Mini kit (Quiagen). A cDNA was synthesized in a 30 µl reaction system from 0.6 µg of the total RNA using TaqMan Reverse Transcription Reagent (Applied Biosystems). PCR was performed in 25 µl of a reaction solution using a buffer attached to an enzyme. The reaction solution contained 1 µl of a template cDNA solution, 0.01 unit of KOD plus DNA polymerase (Toyobo Co., Ltd.), 0.5 µM of a sense primer mPHF3 F2 comprising a partial nucleotide sequence of exon 2 (SEQ ID NO: 9: cccaatttcc agatgccttg), and 0.5 µM of an antisense primer mPHF3 R2 comprising a nucleotide sequence complementary to exon 4 (SEQ ID NO: 10: gcatgcatgt ttgggatcaa). Performance of PCR comprised preincubation at 94°C for 2 minutes, followed by 36 cycles of at 94°C for 15 seconds, at 59°C for 30 seconds and at 68°C for 45 seconds. Results are shown in Fig. 6. In the reactions using the cDNAs from the wild-type mouse lymph nodes, a DNA with a length of 501 bp was amplified. On the other hand, in the cDNAs from the homo-knockout mice, a DNA was not amplified. It was confirmed that expression of the gene is suppressed due to insertion of a trap vector into the genome in the homo-knockout mice.

### Industrial Applicability

According to the present invention can provide a knockout non-human animal whose genome comprises a disruption in at least one allele of the PHF3 gene, more specifically, a knockout non-human animal whose genome comprises a replacement of both or one allele of the diploid PHF3 gene with a function-deficient mutant gene or function-reduced mutant gene thereof, or such a disruption or recombination of both or one allele of the diploid PHF3 gene as to delete or reduce its function, or a progeny animal thereof or a part thereof; a totipotent cell such as an ES cell essential for production of the non-human animal or a part thereof; and a use thereof. The present invention is useful in the field of development of drugs or foods for a disease with the development of atopic dermatitis, and the like.

### Free Text in Sequencing Listing

SEQ ID NO: 3
Designed oligonucleotide primer for PCR

SEQ ID NO: 4
Designed oligonucleotide primer for PCR

SEQ ID NO: 5
Designed oligonucleotide primer for PCR

SEQ ID NO: 6
Designed oligonucleotide primer for PCR

SEQ ID NO: 9
Designed oligonucleotide primer for PCR

SEQ ID NO: 10
Designed oligonucleotide primer for PCR

## Claims

1. A knockout non-human animal whose genome comprises a disruption in at least one allele of the PHF3 gene.

2. The knockout non-human animal according to claim 1, wherein the disruption leads to an insufficient expression of the PHF3 gene.

3. The knockout non-human animal according to claim 1 or 2, wherein the disruption leads to the development of atopic dermatitis.

4. The knockout non-human animal according to any one of claims 1 to 3, wherein the disruption prevents transcription of a full-length mRNA from the allele of the PHF3 gene.

5. The knockout non-human animal according to any one of claims 1 to 4, wherein the disruption prevents transcription from exon 3 and the downstream region thereof in the allele of the PHF3 gene.

6. The knockout non-human animal according to any one of claims 1 to 5, wherein the disruption comprises insertion of a gene cassette.

7. The knockout non-human animal according to claim 6, wherein the gene cassette comprises a nucleotide sequence encoding a selectable marker.

8. The knockout non-human animal according to claim 6, wherein the gene cassette comprises a nucleotide sequence encoding a selectable marker and a transcription termination signal.

9. The knockout non-human animal according to any one of claims 1 to 8, wherein the disruption is a homozygous disruption.

10. The knockout non-human animal according to any one of claims 1 to 9, wherein the non-human animal is a mammal.

11. The knockout non-human animal according to any one of claims 1 to 9, wherein the non-human animal is a rodent.

12. The knockout non-human animal according to any one of claims 1 to 9, wherein the non-human animal is a mouse.

13. The knockout non-human animal according to claim 12, wherein the PHF3 gene comprises a nucleotide sequence encoding the amino acid sequence of SEQ ID NO: 1.

14. The knockout non-human animal according to any one of claims 1 to 13, wherein the disruption leads to representation of one or more properties selected from the group consisting of the following (a) to (d):
(a) high-frequency formation of scabs on the skin as compared with the wild-type non-human animal;
(b) high-frequency cell infiltration into the dermis layer of the skin as compared with the wild-type non-human animal;
(c) a high level of total immunoglobulin E in the blood as compared with the wild-type non-human animal; and
(d) a variation in the expression amount of an atopic dermatitis-associated factor as compared with the wild-type non-human animal.

15. A tissue or cell isolated from the knockout non-human animal according to claim 1.

16. The tissue or cell according to claim 15, which is a T cell, a B cell or a leukocyte-derived cell.

17. A cultured animal cell whose genome comprises a disruption in at least one allele of the PHF3 gene.

18. The cultured animal cell according to claim 17, wherein the disruption leads to an insufficient expression of the PHF3 gene.

19. The cultured animal cell according to claim 17 or 18, which is totipotent.

20. The cultured animal cell according to any one of claims 17 to 19, wherein the PHF3 gene comprises a nucleotide sequence encoding the amino acid sequence of SEQ ID NO: 1.

21. The cultured animal cell according to claim 17 or 18, wherein the PHF3 gene comprises a nucleotide sequence encoding the amino acid sequence of SEQ ID NO: 7.

22. An animal model for a disease with the development of atopic dermatitis, comprising the non-human animal according to claim 1.

23. A method for analyzing a state of a disease associated with the PHF3 protein, which comprises performing on the non-human animal according to claim 1 during the period from the embryonic stage to death:
observation of growth and differentiation, development, and living behavior;
a histopathological test; or
a biochemical test.

24. The analyzing method according to claim 23, wherein the disease associated with the PHF3 protein is a disease with the development of atopic dermatitis.

25. A method for assessing an ability to control atopic dermatitis, which comprises:
(1) the first step of contacting the non-human animal according to claim 1 or a part thereof with a test substance,
(2) the second step of measuring an expression amount of an atopic dermatitis-associated factor in the non-human animal or a part thereof which has been contacted with the test substance in the first step, or an index value correlated with the expression amount, and then comparing the measured value with a control, and
(3) the third step of assessing an ability of the test substance to control atopic dermatitis on the basis of the comparison result of (2).

26. The assessing method according to claim 25, wherein the ability to control atopic dermatitis is an ability to control the expression of an atopic dermatitis-associated factor.

27. The assessing method according to claim 25 or 26, wherein the expression amount of an atopic dermatitis-associated factor is a RNA amount or a protein amount of an atopic dermatitis-associated factor.

28. The assessing method according to any one of claims 25 to 27, wherein the atopic dermatitis associated factor is one or more factors selected from the group consisting of IFN-γ, IL-4, IL-5, IL-13, IL-18 and immunoglobulin E.

29. The assessing method according to claim 28, wherein the atopic dermatitis-associated factor is immunoglobulin E.

30. The assessing method according to any one of claims 25 to 28, wherein the index value correlated with the expression amount of an atopic dermatitis-associated factor is an amount of formed scabs.

31. The assessing method according to any one of claims 25 to 28, wherein the index value correlated with the expression amount of an atopic dermatitis-associated factor is a cell infiltration amount into the dermis layer of the skin.

32. The assessing method according to any one of claims 25 to 28, wherein the index value correlated with the expression amount of an atopic dermatitis-associated factor is the total immunoglobulin E level in the blood.

33. A method for screening an atopoic dermatitis controlling substance, which comprises selecting a test substance having an ability to control atopic dermatitis on the basis of an ability to control atopic dermatitis assessed by the assessing method according to claim 25.

34. A remedy or prophylactic for a disease with the development of atopic dermatitis, which comprises a substance having an ability to control atopic dermatitis obtained by the screening method according to claim 33 as an active ingredient.

35. A recombinant vector for producing the non-human animal according to claim 1 or the cell according to claim 17, wherein the vector carries a gene cassette comprising a selectable marker gene from which a promoter region has been removed, and wherein the vector further comprises a nucleotide sequence homologous to a part of the nucleotide sequence of the PHF3 gene.

36. The recombinant vector according to claim 35, wherein the gene cassette is a gene cassette in which mouse EN2-derived SA (splicing acceptor), encephalomyocarditis Virus-derived IRES (internal ribosome entry site), βgeo in which β-galactosidase and a neomycin resistant gene are fused, and SV40-derived pA (polyadenylation signal) are connected.

37. Use of a substance selected from:
(1) the PHF3 protein or a partial region thereof;
(2) a polynucleotide comprising a nucleotide sequence encoding the amino acid sequence of the protein or a partial region thereof described in (1), or a nucleotide sequence complementary to the nucleotide sequence;
(3) a DNA encoding a RNA having a ribozyme activity which specifically cleaves a nucleotide sequence encoding the amino acid sequence of the protein or a partial region thereof described in (1); and
(4) a DNA encoding a RNA which suppresses the expression of the PHF3 gene on the basis of RNAi effect when expressed in a cell,
for protein therapy or gene therapy of an allergy disease.

38. The use according to claim 37, wherein the PHF3 protein has an activity for ameliorating allergy disease symptoms and comprises any one of the following amino acid sequences:
<Amino acid sequence>
(a) the amino acid sequence of SEQ ID NO: 1 or 7;
(b) the amino acid sequence of SEQ ID NO: 1 or 7 in which one or more amino acids are deleted, added or substituted;
(c) an amino acid sequence with 75% or more sequence identity to the amino acid sequence of SEQ ID NO: 1 or 7;
(d) the amino acid sequence encoded by the nucleotide sequence of SEQ ID NO: 2 or 8;
(e) an amino acid sequence encoded by a DNA comprising a nucleotide sequence with 80% or more sequence identity to the nucleotide sequence of SEQ ID NO: 2 or 8; and
(f) an amino acid sequence encoded by a DNA which hybridizes with a DNA comprising a nucleotide sequence complementary to the nucleotide sequence of SEQ ID NO: 2 or 8 under stringent conditions.

39. An antibody immunologically specific for a protein comprising the amino acid sequence of SEQ ID NO: 1 or 7 or a partial sequence thereof.

40. Use of a substance selected from:
(1) the PHF3 protein or a partial region thereof;
(2) a polynucleotide comprising a nucleotide sequence encoding the amino acid sequence of the protein or a partial region thereof described in (1), or a nucleotide sequence complementary to the nucleotide sequence; and
(3) a gene of the PHF3 protein,
for diagnosing atopic dermatitis.

41. The use according to claim 40, wherein the PHF3 protein has an activity for ameliorating allergy disease symptoms and comprises any one of the following amino acid sequences:
<Amino acid sequence>
(a) the amino acid sequence of SEQ ID NO: 1 or 7;
(b) the amino acid sequence of SEQ ID NO: 1 or 7 in which one or more amino acids are deleted, added or substituted;
(c) an amino acid sequence with 75% or more sequence identity to the amino acid sequence of SEQ ID NO: 1 or 7;
(d) the amino acid sequence encoded by the nucleotide sequence of SEQ ID NO: 2 or 8;
(e) an amino acid sequence encoded by a DNA comprising a nucleotide sequence with 80% or more sequence identity to the nucleotide sequence of SEQ ID NO: 2 or 8; and
(f) an amino acid sequence encoded by a DNA which hybridizes with a DNA comprising a nucleotide sequence complementary to the nucleotide sequence of SEQ ID NO: 2 or 8 under stringent conditions.


SEQUENCE LISTING

<110> SUMITOMO CHEMICAL COMPANY, LIMITED

<110> SUMITOMO PHARMACEUTICALS COMPANY, LIMITED

<110> TRANS GENIC INC.

<120> Knockout non-human animal

<130> S12616W001

<150> JP 2004-233089

<151> 2004-08-10

<160> 10

<210> 1

<211> 2025

<212> PRT

<213> Mus musculus

<400> 1

Met Asp Ile Val Asp Thr Phe Asn His Leu Ile Pro Thr Glu His Leu

1               5                   10                  15

Asp Asp Ala Leu Phe Leu Gly Ser Asn Leu Glu Asn Glu Val Cys Glu

            20                  25                  30

Asp Phe Ser Thr Ser Gln Asn Val Leu Glu Asp Ser Leu Lys Asn Met

        35                  40                  45

Leu Ser Asp Lys Asp Pro Met Leu Gly Ser Ala Ser Asn Gln Phe Cys
    50                  55                  60
Leu Pro Val Leu Asp Ser Asn Asp Pro Asn Phe Gln Met Pro Cys Ser
65                  70                  75                  80
Thr Val Val Gly Leu Asp Asp Ile Met Asp Glu Gly Val Val Lys Glu
                85                  90                  95
Ser Gly Asn Asp Thr Ile Asp Glu Glu Glu Leu Ile Leu Pro Asn Arg
            100                 105                 110
Ser Leu Arg Asp Arg Val Glu Asp Asn Ser Val Arg Ser Pro Arg Lys
        115                 120                 125
Ser Pro Arg Leu Met Ala Gln Glu Gln Val Arg Ser Leu Arg Gln Ser
    130                 135                 140
Thr Ile Ala Lys Arg Ser Asn Ala Ala Thr Leu Ser Thr Lys Lys Pro
145                 150                 155                 160
Ser Gly Lys Thr Leu Ser Thr Ser Lys Val Gly Val Lys Pro Ala Glu
                165                 170                 175
Arg Cys Gln Gly Lys Glu Glu Val Tyr Ala Ser Leu Lys Ser Glu His
            180                 185                 190
Pro Lys Glu Ser Arg Arg Ser Gly Arg His Ala Glu Gln Met Asp Val
        195                 200                 205
Ala Pro Glu Val Ser Ala Ser Ser Val Asp Ser Ser Val Ser Ser Cys
    210                 215                 220
Ala Gly Met Lys Glu Glu Ala Glu Phe Asp Pro Lys His Ala Cys Asn
225                 230                 235                 240
Asn Gln Gly Glu Val Asn Val Pro Ser Pro Glu Leu Asp Cys Pro Leu
                245                 250                 255
Leu Ser Glu Thr Ser Ala Ser Val Glu Glu Lys Asn Ile Glu Ala Leu
            260                 265                 270

Met Glu Cys Lys Ala Lys Thr Asn Ser Ser Pro Leu Phe Lys Phe Pro
        275                 280                 285
Val Arg Glu Asp Glu Gln Ser Asp Leu Val Ser Gly Glu Leu Asn Asp
    290                 295                 300
Thr Ile Glu Gly Lys Asp Ala Gly Gly Lys Pro Asp Gln Glu Ser Glu
305                 310                 315                 320
Glu Val Lys Phe Pro Cys Glu Gly Asp Gln Thr Ala Glu Glu Pro Glu
                325                 330                 335
Ser Ser Asp Val Ser Ser Asp Ser Ala Cys Ala Asn Lys Asn Lys Ala
            340                 345                 350
Glu Lys Asn Glu Gly Ala Glu Cys His Leu Glu Leu Lys Asn Thr Val
        355                 360                 365
Asp Ile Val Asp Lys Pro Glu Asn Ser Pro Gln Arg Asn Glu Leu Glu
    370                 375                 380
Thr Leu Gly Tyr Gly Glu Asp Thr Glu Ser Asn Asp Ala Arg Leu Gln
385                 390                 395                 400
Ser Thr Glu Phe Asn Lys Ser Asp Leu Glu Glu Val Asp Ala Cys Ala
                405                 410                 415
Phe Glu Pro Glu Ala Ser Thr Leu Glu Asn Thr Ile Cys Asp Val Leu
            420                 425                 430
Asp Gln Asn Ser Lys Gln Leu Asn Ile Thr Gln Ser Ile Lys Met Glu
        435                 440                 445
Thr Ala Asn Leu Gln Asp Asp Arg Ser Gly Leu Glu Pro Lys Asn Ile
    450                 455                 460
Lys Pro Lys His Ile Lys Ser Val Thr His Ser Lys Gln Ser Met Thr
465                 470                 475                 480
Thr Glu Thr Pro Arg Lys Thr Val Ala Ala Lys His Glu Val Gly His
                485                 490                 495

Ser Lys Thr Lys Ser Asn Val Lys Ala Val Lys Arg Asn Ser Gly Glu
            500                 505                 510
Pro Glu Pro Gln Pro Asp Ser Gln Arg Pro Val Lys Val Arg Lys Lys
        515                 520                 525
Gln Gly Asp Lys Val Trp Lys Ser Gln Ser Cys Asn Ser Gly Val Lys
    530                 535                 540
Ser Val Lys Ser Gln Ala His Ser Val Leu Lys Arg Met Pro Gln Asp
545                 550                 555                 560
Gln Asn Thr Met Gln Ile Ser Lys Pro Leu Thr His Pro His Ser Asp
                565                 570                 575
Lys Leu His Gly His Ser Gly Phe Ser Lys Glu Pro Pro His Pro Val
            580                 585                 590
Gln Thr Gly His Leu Val His Ser Ser Gln Lys Gln Ser Gln Lys Pro
        595                 600                 605
Gln Gln Gln Ala Ala Val Gly Lys Gly Ser Ser His Val Lys Asp Glu
    610                 615                 620
His Asp His Pro Val Ser Glu His Leu Lys Glu Asp Asp Lys Leu Lys
625                 630                 635                 640
Pro Arg Lys Pro Asp Arg Asn Leu Gln Pro Arg Gln Arg Arg Ser Ser
                645                 650                 655
Arg Ser Phe Ser Leu Asp Glu Pro Pro Leu Phe Ile Pro Asp Asn Ile
            660                 665                 670
Ala Thr Val Lys Lys Glu Gly Ser Asp Gln Thr Thr Ser Ile Glu Ser
        675                 680                 685
Lys Tyr Met Trp Thr Pro Ser Lys Gln Cys Gly Phe Cys Lys Lys Pro
    690                 695                 700
His Gly Asn Arg Phe Met Val Gly Cys Gly Arg Cys Asp Asp Trp Phe
705                 710                 715                 720

His Gly Asp Cys Val Gly Leu Ser Leu Ser Gln Ala Gln Gln Met Gly
                725                 730                 735
Glu Glu Asp Lys Glu Tyr Val Cys Val Arg Cys Cys Ala Glu Glu Asp
            740                 745                 750
Lys Lys Thr Asp Ile Leu Asp Thr Glu Ile Phe Glu Ala Gln Ala Pro
        755                 760                 765
Ile Glu Ala His Ser Glu Asp Lys Arg Met Glu Cys Gly Lys Leu Thr
    770                 775                 780
Ser Ser Lys His Ala Val Thr Asp Asp Lys His Arg His Ser Asp Asp
785                 790                 795                 800
Pro Gly Lys His Lys Val Lys Ile Leu Lys Arg Glu Ser Gly Glu Gly
                805                 810                 815
Lys Thr Ser Ser Asp Ser Arg Asp Asn Glu Ile Lys Lys Trp Gln Leu
            820                 825                 830
Ala Pro Leu Arg Lys Leu Ser Gln Pro His Leu Pro Arg Arg Ser Ser
        835                 840                 845
Glu Glu Lys Ser Glu Lys Ile Ala Lys Glu Ser Thr Ala Leu Ala Ser
    850                 855                 860
Thr Gly Glu Arg Val Ala Arg Ser Gly Thr His Glu Lys Gln Glu Thr
865                 870                 875                 880
Lys Lys Lys Lys Met Glu Lys Gly Gly Pro Asn Val His Pro Pro Ala
                885                 890                 895
Ala Thr Ser Lys Pro Ser Ala Asp Gln Ile Arg Gln Ser Val Arg His
            900                 905                 910
Ser Leu Lys Asp Ile Leu Met Lys Arg Leu Thr Asp Ser Asn Leu Lys
        915                 920                 925
Ile Pro Glu Glu Lys Ala Ala Lys Val Ala Thr Lys Ile Glu Lys Glu
    930                 935                 940

Leu Phe Ser Phe Phe Arg Asp Thr Asp Ala Lys Tyr Lys Asn Lys Tyr
945                 950                 955                 960
Arg Ser Leu Met Phe Asn Leu Lys Asp Pro Lys Asn Asn Ile Leu Phe
                965                 970                 975
Lys Lys Val Leu Lys Gly Glu Val Thr Pro Asp His Leu Ile Arg Met
            980                 985                 990
Ser Pro Glu Glu Leu Ala Ser Lys Glu Leu Ala Ala Trp Arg Arg Arg
        995                 1000                1005
Glu Asn Arg His Thr Ile Glu Met Ile Glu Lys Glu Gln Arg Glu Val
    1010                1015                1020
Glu Arg Arg Pro Ile Thr Lys Ile Thr His Lys Gly Glu Ile Glu Ile
1025                1030                1035                1040
Glu Ser Asp Ala Pro Met Lys Glu Gln Glu Ala Ala Ile Glu Ile Gln
                1045                1050                1055
Glu Pro Ser Ala Asn Lys Ser Met Glu Lys Pro Asp Val Ser Glu Lys
            1060                1065                1070
Gln Lys Glu Glu Val Asp Ser Thr Ser Lys Asp Thr Thr Ser Gln His
        1075                1080                1085
Arg Gln His Leu Phe Asp Leu Asn Cys Lys Ile Cys Ile Gly Arg Met
    1090                1095                1100
Ala Pro Pro Ile Asp Asp Leu Ser Pro Lys Thr Val Lys Val Val Val
1105                1110                1115                1120
Gly Gly Ala Arg Lys His Ser Asp Asn Glu Ala Glu Ser Leu Ala Asp
                1125                1130                1135
Ala Leu Ser Ser Thr Thr Asn Ile Leu Thr Ser Asp Leu Phe Glu Glu
            1140                1145                1150
Glu Lys Gln Glu Ser Pro Lys Ser Thr Phe Ser Pro Thr Pro Arg Pro
        1155                1160                1165

Glu Met Pro Gly Thr Val Glu Val Glu Ser Thr Phe Leu Ala Arg Leu
    1170                1175                1180
Asn Phe Ile Trp Lys Gly Phe Ile Asn Met Pro Ser Val Ala Lys Phe
1185                1190                1195                1200
Val Thr Lys Ala Tyr Pro Val Ser Gly Ser Pro Glu Tyr Leu Thr Glu
                1205                1210                1215
Asp Leu Pro Asp Ser Ile Gln Val Gly Gly Arg Ile Ser Pro Gln Thr
            1220                1225                1230
Val Trp Asp Tyr Val Glu Lys Ile Lys Ala Ser Gly Thr Lys Glu Ile
        1235                1240                1245
Cys Val Val Arg Phe Thr Pro Val Thr Glu Glu Asp Gln Ile Ser Tyr
    1250                1255                1260
Thr Leu Leu Phe Ala Tyr Phe Ser Ser Arg Lys Arg Tyr Gly Val Ala
1265                1270                1275                1280
Ala Asn Asn Met Lys Gln Val Lys Asp Met Tyr Leu Ile Pro Leu Gly
                1285                1290                1295
Ala Ala Asp Lys Ile Pro His Pro Leu Val Pro Phe Asp Gly Pro Gly
            1300                1305                1310
Leu Glu Leu His Arg Pro Asn Leu Leu Leu Gly Leu Ile Ile Arg Gln
        1315                1320                1325
Lys Leu Lys Arg Pro His Ser Ala Ser Ala Gly Pro Ser His Thr Gly
    1330                1335                1340
Glu Thr Pro Glu Ser Ala Pro Ile Val Leu Pro Pro Asp Lys Lys Gly
1345                1350                1355                1360
Lys Met Glu Ser Cys Thr Glu Glu Ala Ala Glu Glu Glu Ser Asp Phe
                1365                1370                1375
Phe Asn Ser Phe Thr Thr Val Leu His Lys Gln Arg Asn Lys Pro Ser
            1380                1385                1390

Gln Pro Leu Gln Glu Asp Leu Pro Thr Ala Ala Glu Pro Leu Met Glu
        1395                1400                1405
Val Thr Lys Gln Glu Pro Pro Lys Pro Leu Arg Phe Leu Pro Gly Val
    1410                1415                1420
Leu Ile Gly Trp Asp Asn Gln Pro Ser Thr Leu Glu Leu Ala Asn Lys
1425                1430                1435                1440
Pro Leu Pro Val Asp Asp Ile Leu Gln Ser Leu Leu Gly Thr Thr Gly
                1445                1450                1455
Gln Val Tyr Glu Gln Ala Gln Pro Leu Val Glu Gln Ser Thr Leu Lys
            1460                1465                1470
Glu Ile Pro Phe Ile Asn Asp Gln Ala Asn Pro Lys Val Glu Lys Ile
        1475                1480                1485
Asp Lys Val Glu Val Thr Glu Gly Asp Ala Lys Glu Ile Lys Val Lys
    1490                1495                1500
Ala Glu Asn Ile Ser Val Ser Thr Ser Lys Asn Ser Gly Glu Glu Thr
1505                1510                1515                1520
Ser Ser Val Gly Ser Ser Ser Ile Ser Pro Gly Pro Leu Ala Ser Leu
                1525                1530                1535
Ser Leu Arg Gly Lys Pro Pro Asp Val Ser Thr Glu Ala Phe Leu Thr
            1540                1545                1550
Asn Leu Ser Ile Pro Ser Lys Gln Glu Glu Ser Val Glu Asn Lys Glu
        1555                1560                1565
Arg Thr Leu Lys Arg Leu Leu Leu Gln Asp Gln Glu Asn Ser Leu Gln
    1570                1575                1580
Asp Asn Arg Thr Ser Ser Asp Ser Pro Cys Trp Pro Gly Thr Gly Lys
1585                1590                1595                1600
Gly Gly Met Asp Gly Asp Gly Ser Gly Ser Gly Ser Gly Ser Gly Ser
                1605                1610                1615

Gly Ser Glu Gly Pro Val Ala Asn Thr Arg Ala Pro Gln Phe Ile Asn
            1620                1625                1630
Leu Lys Arg Asp Pro Arg Gln Ala Ala Gly Arg Ser Gln Gln Thr Ala
        1635                1640                1645
Ser Glu Ser Lys Asp Ala Glu Ser Cys Arg Asn Gly Asp Lys His Ala
    1650                1655                1660
Ala Ser Ala Pro Pro His Asn Lys Glu Pro Leu Ala Glu Ala Val Gly
1665                1670                1675                1680
Gly Glu Gly Lys Leu Pro Ser Gln Glu Lys Ser Ser Cys Val Glu Gln
                1685                1690                1695
Asn Asp Asp Ser Glu Ala Ala Pro Asn Ser Ser Ser Val Glu Asn Leu
            1700                1705                1710
Asn Ser Ser Gln Ala Glu Gln Ala Asn Pro Ser Gln Glu Asp Val Leu
        1715                1720                1725
Thr Gln Asn Ile Glu Thr Val His Pro Phe Arg Arg Gly Ser Ala Pro
    1730                1735                1740
Thr Ser Ser Arg Phe Glu Gly Gly Asn Thr Cys Gln Ser Glu Phe Pro
1745                1750                1755                1760
Ser Lys Ser Val Ser Phe Thr Cys Arg Ser Ser Ser Pro Arg Ala Ser
                1765                1770                1775
Thr Asn Phe Ser Pro Met Arg Pro Gln Gln Pro Asn Leu Gln His Leu
            1780                1785                1790
Lys Ser Ser Pro Pro Gly Phe Pro Phe Pro Gly Pro Gln Asn Phe Pro
        1795                1800                1805
Pro Gln Asn Met Phe Gly Phe Pro Pro His Leu Ser Pro Pro Leu Leu
    1810                1815                1820
Pro Pro Pro Gly Phe Gly Phe Pro Gln Asn Pro Pro Met Val Pro Trp
1825                1830                1835                1840

Pro Pro Val His Val Pro Gly Gln Pro Gln Arg Met Met Gly Pro Leu
                1845                1850                1855
Ser Gln Ala Ser Arg Tyr Met Gly Pro Gln Asn Phe Tyr Gln Val Lys
            1860                1865                1870
Asp Ile Arg Arg Pro Glu Arg Arg His Ser Asp Pro Trp Gly Arg Gln
        1875                1880                1885
Asp Gln Gln Gln Pro Asp Arg Pro Phe Asn Arg Gly Lys Gly Asp Arg
    1890                1895                1900
Gln Arg Phe Tyr Ser Asp Ser His His Leu Lys Arg Glu Arg His Asp
1905                1910                1915                1920
Lys Asp Trp Glu Gln Glu Ser Glu Arg His Arg His Arg Asp Arg Ser
                1925                1930                1935
Gln Glu Arg Asp Arg Asp Arg Lys Ser Lys Glu Glu Ala Ala Ala His
            1940                1945                1950
Lys Asp Lys Glu Arg Pro Arg Leu Ser His Gly Asp Arg Ala Pro Asp
        1955                1960                1965
Gly Lys Ala Ser Arg Asp Gly Lys Ser Ala Asp Lys Lys Pro Asp Arg
    1970                1975                1980
Pro Lys Gly Glu Asp His Glu Lys Glu Lys Glu Arg Asp Lys Ser Lys
1985                1990                1995                2000
His Lys Glu Gly Glu Lys Asp Arg Glu Arg Tyr His Lys Asp Arg Asp
                2005                2010                2015
His Thr Asp Arg Val Lys Ser Lys Arg
            2020                2025

<210> 2
<211> 7111

<212> DNA

<213> Mus musculus

<400> 2

gtgacgcggc cccgctcctg ctcccgcgcc gccgccgccg caagccgatg gctgcgggct 60

ctcgcggcgc cgcacaggct ccacgaggcg agcgaccccc gggctcaggg aagcagcgac 120

tgcagctcgg acggggaggg cggagcgcag gccggccgta gtgtcgtcac cgcggcggcg 180

cgggccgggg agccccgagc ccaggcagca acgccgacgc cctgcgcgca ccccctccgc 240

ggccccctcc tccgcggccc cgcggcctcc atcttcctct ggctgccggt ggcagcgctc 300

gtctgcggag cggggttgaa agacacacag aagtcttcat ggatatagtt gatacattta 360

atcatttaat tcctactgaa cacttagatg atgccctatt tctaggatcc aacctggaga 420

atgaagtctg tgaggatttt agtacaagtc aaaatgtctt agaggactcg ctgaagaaca 480

tgctcagcga taaggatcct atgctaggat ctgcaagtaa ccagttctgt ttgcctgttt 540

tggatagcaa tgatcccaat ttccagatgc cttgttcaac agttgttggt cttgacgata 600

ttatggatga aggagttgtt aaagaaagtg gtaatgatac cattgatgaa gaagaattga 660

ttttacctaa caggagtttg agggacagag tagaagacaa ttcagtaaga tcaccaagaa 720

aatcacctcg tttaatggca caagaacaag taagaagttt gcgacaaagc actattgcca 780

agcgttcaaa tgcagcaact ctaagcacaa aaaagccatc tgggaagacc ttatctacct 840

ctaaagtagg ggtgaagcca gcagaaagat gtcagggtaa agaagaagtt tatgcctcac 900

tgaaatctga acaccctaag gagagtagaa ggagtggccg gcatgctgaa cagatggacg 960

tggcgccaga agtctcagca tcttcagttg attcttcagt gtcgtcttgt gctggaatga 1020

aggaggaggc tgagtttgat cccaaacatg catgcaataa tcagggtgaa gtgaacgtgc 1080

catctcctga gttagattgt ccacttctct cagagactag tgctagtgtg gaagagaaga 1140

acattgaggc tctgatggaa tgtaaagcta agactaacag tagcccgtta tttaagtttc 1200

cagttagaga agatgagcag agtgatcttg tttcgggtga actgaatgac accattgaag 1260

gaaaggatgc agggggaaaa cctgatcaag aatcagagga ggtgaagttt ccctgcgaag 1320

gtgaccagac ggccgaggaa cccgaatctt ctgatgtgtc tagtgactct gcttgtgcca 1380

ataaaaataa ggcagaaaag aatgaagggg cagaatgtca tttggaattg aagaatactg 1440

tggatattgt tgataaacct gagaactcac ctcagagaaa tgagttggaa acactgggct 1500
atggtgagga cacggagtct aatgatgctc ggttacagag cacagagttc aataagtcag 1560
atttggagga agttgatgct tgtgcttttg aaccggaagc tagcacttta gaaaatacta 1620
tttgtgatgt tcttgaccaa aattcaaagc aattgaatat tactcaaagt attaaaatgg 1680
aaacagccaa tcttcaggat gatagaagtg gtttagagcc caaaaacata aaacccaaac 1740
acataaaatc tgtaactcat tctaaacaaa gcatgaccac agagacaccc agaaaaactg 1800
tggcagcaaa gcatgaagta ggtcatagca aaactaaatc taatgtcaag gctgtgaagc 1860
gaaattctgg tgagccggaa ccccagcctg attctcagag gccagtcaaa gtgagaaaaa 1920
aacaaggtga taaggtttgg aagagtcaga gttgtaattc tggagttaaa tctgtgaaaa 1980
gccaagctca ttctgtattg aaaagaatgc cccaggatca aaacacaatg cagatttcca 2040
aacctttaac tcatcctcat agtgataagc ttcatggtca ctctggcttc tctaaggagc 2100
ctccccatcc tgtacaaact ggacacttgg tgcattccag tcaaaaacag agccagaagc 2160
ctcagcagca ggcagctgtg gggaagggca gtagccatgt gaaggatgag catgaccatc 2220
cagtcagtga gcatcttaag gaggacgata aactgaagcc aagaaaacct gataggaacc 2280
tgcagcctcg ccaaaggaga agcagccgaa gtttttctct ggatgagcct ccattgttca 2340
tcccagacaa catagctact gtaaaaaaag aagggtcaga tcagaccacc tcaattgaaa 2400
gcaaatatat gtggactcca agcaagcaat gtgggttttg caaaaaacca catggcaaca 2460
ggtttatggt cggctgtggg agatgtgatg actggtttca tggagactgt gttggattaa 2520
gtctttctca agcacagcaa atgggagagg aagacaaaga atatgtttgt gtaaggtgct 2580
gtgctgaaga agataaaaag actgacatac tagacacaga gatttttgaa gcccaagctc 2640
ccatcgaagc ccacagcgaa gataaaagga tggagtgtgg aaagctgaca tcatcgaagc 2700
acgcagtcac tgacgataaa cacagacact cagatgaccc tgggaagcac aaggtcaaaa 2760
ttttaaaacg ggagtctggt gagggaaaaa cttcctctga cagtagagat aatgaaatta 2820
aaaaatggca gctagcccct cttcgcaagt tgagccaacc acatttacct cggagatcat 2880
cagaagaaaa aagtgaaaaa atagcaaagg aatctacagc tcttgcctct acaggagaga 2940
gagtggcgag atcaggtact catgagaagc aagagacaaa aaagaagaaa atggaaaagg 3000
gtggacctaa tgtgcacccg cctgctgcta cttccaagcc ttctgcagat cagatcagac 3060
agagtgtccg acattctctc aaagatattc ttatgaaaag acttacagat tcaaacctga 3120

agatcccaga ggaaaaggca gcaaaggttg ccacaaaaat tgaaaaagaa cttttctctt 3180
tttttcggga cacggatgcc aagtataaga ataaatatag aagcttgatg tttaatctaa 3240
aagatcctaa aaacaatata ttatttaaga aagttctaaa aggagaagta acccctgatc 3300
atcttataag aatgagccca gaagaactag cctccaaaga gctggctgcg tggagacgaa 3360
gggagaacag acacaccata gagatgattg agaaggaaca gagagaagtg gaaagacgac 3420
caatcacgaa aataactcac aaaggtgaga tagaaatcga gagtgacgcc ccgatgaagg 3480
agcaggaagc agccatcgag atccaggaac cgtcagctaa taagtcaatg gagaagccag 3540
atgtgtctga gaaacaaaaa gaagaggttg actccacatc taaagacacc actagtcaac 3600
acagacagca tctctttgac ctgaactgca aaatatgcat aggtcgaatg gcaccaccta 3660
tagatgatct ttctccaaaa actgtgaaag ttgttgtagg aggagcccgg aaacactcag 3720
acaacgaagc agagagtcta gcagatgcgt tgtcttcaac tacgaatatt ctgacctctg 3780
acctctttga ggaggaaaag caagaatctc ccaagtcaac gttttccccc actccacggc 3840
cagagatgcc tggaactgtg gaagttgagt caacctttct ggctcgattg aacttcatct 3900
ggaaaggttt tatcaatatg ccctctgtgg caaagtttgt taccaaagcc taccccgtgt 3960
ctggatcccc tgagtacttg acagaggacc taccagacag cattcaagta ggtggcagga 4020
tatcgcctca gacagtttgg gattatgtgg aaaaaataaa agcatcaggc accaaggaaa 4080
tctgtgtggt tcgcttcaca ccagtaactg aagaagatca gatttcttac actttgctgt 4140
ttgcgtactt cagtagcaga aagcgctatg gtgtggctgc taacaacatg aagcaggtta 4200
aagacatgta cctcattcca ttgggcgctg cagataaaat cccacaccct cttgtgcctt 4260
ttgatggacc tggacttgag ctgcacagac ctaatctgtt gttgggcctg attatccgtc 4320
agaagctgaa gcggccgcac agcgctagtg caggccccag tcacacaggc gagactcctg 4380
agagcgcccc aatagtgttg ccccccgaca agaaaggtaa aatggagtca tgcacagagg 4440
aagcagcaga ggaagagagt gactttttca attcctttac aactgtgtta cacaagcaga 4500
gaaataagcc ctcacagcct cttcaggaag acctgccaac agcagctgaa cctctgatgg 4560
aagtcaccaa gcaggagcca ccaaaaccct taaggttcct tcctggggtc ctgattggct 4620
gggacaatca accttctact ctggaattag caaataagcc tcttcctgtg gatgacatac 4680
tccaaagcct tttgggcacc actggtcaag tatatgagca ggctcagcca cttgtagaac 4740
aaagcactct taaagaaatt ccttttataa atgatcaagc taacccaaaa gtagagaaaa 4800

tagataaagt ggaggtgact gaaggtgacg ccaaggagat aaaagttaaa gcagaaaata 4860
tttcagtgtc tacaagtaaa aactcaggag aagagacttc atcggtaggt tcatcttcca 4920
tttctccagg gcctttggca agtctcagtc tgagagggaa accaccagat gtttctacag 4980
aagcattctt aacaaattta tcaattccct caaaacaaga ggaaagtgtg gaaaacaaag 5040
agagaacatt aaaaagactg ctgctccaag atcaagagaa tagtttgcaa gacaatagga 5100
cttcaagtga ctctccctgc tggcctggca ctgggaaggg aggcatggat ggggatggga 5160
gcgggagcgg gagcgggagc gggagcggca gtgagggtcc agtggctaac acaagagccc 5220
cacagtttat caacctgaaa agggacccta ggcaagcagc aggacgaagt cagcagacag 5280
cttctgaaag caaggatgca gagagctgca gaaatggaga caagcatgct gcgtctgctc 5340
caccacacaa caaagagccc ttagcagagg ctgtcggtgg agagggaaag ctgccttcgc 5400
aggagaagag ctcatgtgta gagcagaacg acgattcgga agctgcacca aactcatcct 5460
cagtagaaaa cttaaattcg tcacaagcag aacaagccaa tccttcacaa gaggatgttt 5520
taacacaaaa tattgaaacg gtccacccgt ttagaagagg atcggctcca acatcatctc 5580
gttttgaagg tggaaatacc tgccaatcag aatttccgtc taaaagcgtt agctttactt 5640
gcaggagtag cagccctcgc gcgagtacaa acttttcacc tatgaggcca cagcagccca 5700
atctccagca cctcaagtcc agtcctcctg gattcccatt tccaggaccc caaaacttcc 5760
ctccgcaaaa tatgtttgga ttcccaccac atttgtcacc tccgttactt ccccctccag 5820
gcttcggctt ccctcaaaat ccccccatgg ttccttggcc acctgttcat gtcccaggcc 5880
aaccacaacg catgatgggg cccctttcac aagcatcacg gtacatgggc ccacaaaatt 5940
tttatcaggt aaaagatatt cggagaccag aaaggcgcca tagtgaccca tggggtaggc 6000
aagaccaaca acaaccagat aggccattta ataggggtaa aggggaccga cagagatttt 6060
acagtgattc gcaccacttg aaaagagagc gtcacgacaa ggattgggag caggaatccg 6120
aaaggcacag acacagagac agaagccaag aaagggacag agacagaaaa agcaaagagg 6180
aagcagcggc acacaaagac aaggagagac cgcggctctc acacggcgac cgggcaccag 6240
atgggaaagc aagcagagat ggtaagagtg cagacaagaa accggacagg cctaaaggcg 6300
aagaccacga aaaggagaaa gagagagaca agagcaagca caaagaaggg gagaaggaca 6360
gagagcggta ccacaaagac agggaccaca ccgacagagt gaaaagcaaa aggtgaaact 6420
ggaggctgct ttagcgtctt acatgtgtgt ctttataaga ttgcctggta ggactgtgcc 6480

atcttaaccc tcggattgct gcttggcaga gcagtaaact atgctggtgc acggtcctgt 6540

gaccagtgcc ctccgcccct tctttacacc actgtcctac ctacaggaat ttaatatttt 6600

taaagtttta caatactgta tattcaaaga tttgtttaat taatatgcaa taaaggctta 6660

gaaattttag ttttattcct taattggtaa atatggttaa ctatgaaata tatttcctgc 6720

ctctagtgaa tgtcctttct ataatgacta atttgaaagt aatctgtgct ctgtacattt 6780

gttttcaatt gcactgtttt taaagaaaat gtagaggagc agcaaaaaag ccaagcgact 6840

tcccgatgag accatactat ttaggtggca gtttttgact gtatcacgac cccagggagg 6900

tcatgggttg ctttaatctg tgctcattga aggcgtttct taccatgtac tgcagtttgt 6960

ggtaggccat atccctttcc tttctggctc ttcagaaact tgaatactta agcttgtaca 7020

tgatcttgtg ttttgctatc ctttttactg taaaatgtaa atattttaag ggatattttg 7080

attctaaata tgataaaaga atttctcacc t 7111

<210> 3

<211> 22

<212> DNA

<213> Artificial sequence

<220>

<223> Designed oligonucleotide primer for PCR

<400> 3

gtggcaatga taccattgat ga 22

<210> 4

<211> 23

<212> DNA

<213> Artificial sequence

<220>
<223> Designed oligonucleotide primer for PCR

<400> 4
cacttcatct acacaactag gca          23

<210> 5
<211> 23
<212> DNA
<213> Artificial sequence

<220>
<223> Designed oligonucleotide primer for PCR

<400> 5
accaactggg acgacatgga gaa          23

<210> 6
<211> 20
<212> DNA
<213> Artificial sequence

<220>
<223> Designed oligonucleotide primer for PCR

<400> 6

gtggtggtga agctgtagcc        20

<210> 7

<211> 2039

<212> PRT

<213> Homo sapiens

<400> 7

Met Asp Ile Val Asp Thr Phe Asn His Leu Ile Pro Thr Glu His Leu

1               5                   10                  15

Asp Asp Ala Leu Phe Leu Gly Ser Asn Leu Glu Asn Glu Val Cys Glu

            20                  25                  30

Asp Phe Ser Ala Ser Gln Asn Val Leu Glu Asp Ser Leu Lys Asn Met

        35                  40                  45

Leu Ser Asp Lys Asp Pro Met Leu Gly Ser Ala Ser Asn Gln Phe Cys

    50                  55                  60

Leu Pro Val Leu Asp Ser Asn Asp Pro Asn Phe Gln Met Pro Cys Ser

65                  70                  75                  80

Thr Val Val Gly Leu Asp Asp Ile Met Asp Glu Gly Val Val Lys Glu

                85                  90                  95

Ser Gly Asn Asp Thr Ile Asp Glu Glu Glu Leu Ile Leu Pro Asn Arg

            100                 105                 110

Asn Leu Arg Asp Lys Val Glu Glu Asn Ser Val Arg Ser Pro Arg Lys

        115                 120                 125

Ser Pro Arg Leu Met Ala Gln Glu Gln Val Arg Ser Leu Arg Gln Ser

    130                 135                 140
Thr Ile Ala Lys Arg Ser Asn Ala Ala Pro Leu Ser Asn Thr Lys Lys
145                 150                 155                 160
Ala Ser Gly Lys Thr Val Ser Thr Ala Lys Ala Gly Val Lys Gln Pro
                165                 170                 175
Glu Arg Ser Gln Val Lys Glu Glu Val Cys Met Ser Leu Lys Pro Glu
            180                 185                 190
Tyr His Lys Glu Asn Arg Arg Cys Ser Arg Asn Ser Gly Gln Ile Glu
        195                 200                 205
Val Val Pro Glu Val Ser Val Ser Ser Ser His Ser Ser Val Ser Ser
    210                 215                 220
Cys Leu Glu Met Lys Asp Glu Asp Gly Leu Asp Ser Lys His Lys Cys
225                 230                 235                 240
Asn Asn Pro Gly Glu Ile Asp Val Pro Ser His Glu Leu Asn Cys Ser
                245                 250                 255
Leu Leu Ser Glu Thr Cys Val Thr Ile Gly Glu Lys Lys Asn Glu Ala
            260                 265                 270
Leu Met Glu Cys Lys Ala Lys Pro Val Gly Ser Pro Leu Phe Lys Phe
        275                 280                 285
Ser Asp Lys Glu Glu His Glu Gln Asn Asp Ser Ile Ser Gly Lys Thr
    290                 295                 300
Gly Glu Thr Val Val Glu Glu Met Ile Ala Thr Arg Lys Val Glu Gln
305                 310                 315                 320
Asp Ser Lys Glu Thr Val Lys Leu Ser His Glu Asp Asp His Ile Leu
                325                 330                 335
Glu Asp Ala Gly Ser Ser Asp Ile Ser Ser Asp Ala Ala Cys Thr Asn
            340                 345                 350
Pro Asn Lys Thr Glu Asn Ser Leu Val Gly Leu Pro Ser Cys Val Asp

        355                 360                 365
Glu Val Thr Glu Cys Asn Leu Glu Leu Lys Asp Thr Met Gly Ile Ala
    370                 375                 380
Asp Lys Thr Glu Asn Thr Leu Glu Arg Asn Lys Ile Glu Pro Leu Gly
385                 390                 395                 400
Tyr Cys Glu Asp Ala Glu Ser Asn Arg Gln Leu Glu Ser Thr Glu Phe
                405                 410                 415
Asn Lys Ser Asn Leu Glu Val Val Asp Thr Ser Thr Phe Gly Pro Glu
            420                 425                 430
Ser Asn Ile Leu Glu Asn Ala Ile Cys Asp Val Pro Asp Gln Asn Ser
        435                 440                 445
Lys Gln Leu Asn Ala Ile Glu Ser Thr Lys Ile Glu Ser His Glu Thr
    450                 455                 460
Ala Asn Leu Gln Asp Asp Arg Asn Ser Gln Ser Ser Ser Val Ser Tyr
465                 470                 475                 480
Leu Glu Ser Lys Ser Val Lys Ser Lys His Thr Lys Pro Val Ile His
                485                 490                 495
Ser Lys Gln Asn Met Thr Thr Asp Ala Pro Lys Lys Ile Val Ala Ala
            500                 505                 510
Lys Tyr Glu Val Ile His Ser Lys Thr Lys Val Asn Val Lys Ser Val
        515                 520                 525
Lys Arg Asn Thr Asp Val Pro Glu Ser Gln Gln Asn Phe His Arg Pro
    530                 535                 540
Val Lys Val Arg Lys Lys Gln Ile Asp Lys Glu Pro Lys Ile Gln Ser
545                 550                 555                 560
Cys Asn Ser Gly Val Lys Ser Val Lys Asn Gln Ala His Ser Val Leu
                565                 570                 575
Lys Lys Thr Leu Gln Asp Gln Thr Leu Val Gln Ile Phe Lys Pro Leu

            580                 585                 590
Thr His Ser Leu Ser Asp Lys Ser His Ala His Pro Gly Cys Leu Lys
        595                 600                 605
Glu Pro His His Pro Ala Gln Thr Gly His Val Ser His Ser Ser Gln
    610                 615                 620
Lys Gln Cys His Lys Pro Gln Gln Gln Ala Pro Ala Met Lys Thr Asn
625                 630                 635                 640
Ser His Val Lys Glu Glu Leu Glu His Pro Gly Val Glu His Phe Lys
                645                 650                 655
Glu Glu Asp Lys Leu Lys Leu Lys Lys Pro Glu Lys Asn Leu Gln Pro
            660                 665                 670
Arg Gln Arg Arg Ser Ser Lys Ser Phe Ser Leu Asp Glu Pro Pro Leu
        675                 680                 685
Phe Ile Pro Asp Asn Ile Ala Thr Ile Arg Arg Glu Gly Ser Asp His
    690                 695                 700
Ser Ser Ser Phe Glu Ser Lys Tyr Met Trp Thr Pro Ser Lys Gln Cys
705                 710                 715                 720
Gly Phe Cys Lys Lys Pro His Gly Asn Arg Phe Met Val Gly Cys Gly
                725                 730                 735
Arg Cys Asp Asp Trp Phe His Gly Asp Cys Val Gly Leu Ser Leu Ser
            740                 745                 750
Gln Ala Gln Gln Met Gly Glu Glu Asp Lys Glu Tyr Val Cys Val Lys
        755                 760                 765
Cys Cys Ala Glu Glu Asp Lys Lys Thr Glu Ile Leu Asp Pro Asp Thr
    770                 775                 780
Leu Glu Asn Gln Ala Thr Val Glu Phe His Ser Gly Asp Lys Thr Met
785                 790                 795                 800
Glu Cys Glu Lys Leu Gly Leu Ser Lys His Thr Thr Asn Asp Arg Thr

                805                 810                 815
Lys Tyr Ile Asp Asp Thr Val Lys His Lys Val Lys Ile Leu Lys Arg
            820                 825                 830
Glu Ser Gly Glu Gly Arg Asn Ser Ser Asp Cys Arg Asp Asn Glu Ile
        835                 840                 845
Lys Lys Trp Gln Leu Ala Pro Leu Arg Lys Met Gly Gln Pro Val Leu
    850                 855                 860
Pro Arg Arg Ser Ser Glu Glu Lys Ser Glu Lys Ile Pro Lys Glu Ser
865                 870                 875                 880
Thr Thr Val Thr Cys Thr Gly Glu Lys Ala Ser Lys Pro Gly Thr His
                885                 890                 895
Glu Lys Gln Glu Met Lys Lys Lys Lys Val Glu Lys Gly Val Leu Asn
            900                 905                 910
Val His Pro Ala Ala Ser Ala Ser Lys Pro Ser Ala Asp Gln Ile Arg
        915                 920                 925
Gln Ser Val Arg His Ser Leu Lys Asp Ile Leu Met Lys Arg Leu Thr
    930                 935                 940
Asp Ser Asn Leu Lys Val Pro Glu Glu Lys Ala Ala Lys Val Ala Thr
945                 950                 955                 960
Lys Ile Glu Lys Glu Leu Phe Ser Phe Phe Arg Asp Thr Asp Ala Lys
                965                 970                 975
Tyr Lys Asn Lys Tyr Arg Ser Leu Met Phe Asn Leu Lys Asp Pro Lys
            980                 985                 990
Asn Asn Ile Leu Phe Lys Lys Val Leu Lys Gly Glu Val Thr Pro Asp
        995                 1000                1005
His Leu Ile Arg Met Ser Pro Glu Glu Leu Ala Ser Lys Glu Leu Ala
    1010                1015                1020
Ala Trp Arg Arg Arg Glu Asn Arg His Thr Ile Glu Met Ile Glu Lys

025                 1030                1035                1040
Glu Gln Arg Glu Val Glu Arg Arg Pro Ile Thr Lys Ile Thr His Lys
                1045                1050                1055
Gly Glu Ile Glu Ile Glu Ser Asp Ala Pro Met Lys Glu Gln Glu Ala
            1060                1065                1070
Ala Met Glu Ile Gln Glu Pro Ala Ala Asn Lys Ser Leu Glu Lys Pro
        1075                1080                1085
Glu Gly Ser Glu Lys Gln Lys Glu Glu Val Asp Ser Met Ser Lys Asp
    1090                1095                1100
Thr Thr Ser Gln His Arg Gln His Leu Phe Asp Leu Asn Cys Lys Ile
1105                1110                1115                1120
Cys Ile Gly Arg Met Ala Pro Pro Val Asp Asp Leu Ser Pro Lys Lys
                1125                1130                1135
Val Lys Val Val Val Gly Val Ala Arg Lys His Ser Asp Asn Glu Ala
            1140                1145                1150
Glu Ser Ile Ala Asp Ala Leu Ser Ser Thr Ser Asn Ile Leu Ala Ser
        1155                1160                1165
Glu Phe Phe Glu Glu Glu Lys Gln Glu Ser Pro Lys Ser Thr Phe Ser
    1170                1175                1180
Pro Ala Pro Arg Pro Glu Met Pro Gly Thr Val Glu Val Glu Ser Thr
1185                1190                1195                1200
Phe Leu Ala Arg Leu Asn Phe Ile Trp Lys Gly Phe Ile Asn Met Pro
                1205                1210                1215
Ser Val Ala Lys Phe Val Thr Lys Ala Tyr Pro Val Ser Gly Ser Pro
            1220                1225                1230
Glu Tyr Leu Thr Glu Asp Leu Pro Asp Ser Ile Gln Val Gly Gly Arg
        1235                1240                1245
Ile Ser Pro Gln Thr Val Trp Asp Tyr Val Glu Lys Ile Lys Ala Ser

1250                1255                1260
Gly Thr Lys Glu Ile Cys Val Val Arg Phe Thr Pro Val Thr Glu Glu
1265                1270                1275                1280
Asp Gln Ile Ser Tyr Thr Leu Leu Phe Ala Tyr Phe Ser Ser Arg Lys
                1285                1290                1295
Arg Tyr Gly Val Ala Ala Asn Asn Met Lys Gln Val Lys Asp Met Tyr
            1300                1305                1310
Leu Ile Pro Leu Gly Ala Thr Asp Lys Ile Pro His Pro Leu Val Pro
        1315                1320                1325
Phe Asp Gly Pro Gly Leu Glu Leu His Arg Pro Asn Leu Leu Leu Gly
    1330                1335                1340
Leu Ile Ile Arg Gln Lys Leu Lys Arg Gln His Ser Ala Cys Ala Ser
1345                1350                1355                1360
Thr Ser His Ile Ala Glu Thr Pro Glu Ser Ala Pro Pro Ile Ala Leu
                1365                1370                1375
Pro Pro Asp Lys Lys Ser Lys Ile Glu Val Ser Thr Glu Glu Ala Pro
            1380                1385                1390
Glu Glu Glu Asn Asp Phe Phe Asn Ser Phe Thr Thr Val Leu His Lys
        1395                1400                1405
Gln Arg Asn Lys Pro Gln Gln Asn Leu Gln Glu Asp Leu Pro Thr Ala
    1410                1415                1420
Val Glu Pro Leu Met Glu Val Thr Lys Gln Glu Pro Pro Lys Pro Leu
1425                1430                1435                1440
Arg Phe Leu Pro Gly Val Leu Ile Gly Trp Glu Asn Gln Pro Thr Thr
                1445                1450                1455
Leu Glu Leu Ala Asn Lys Pro Leu Pro Val Asp Asp Ile Leu Gln Ser
            1460                1465                1470
Leu Leu Gly Thr Thr Gly Gln Val Tyr Asp Gln Ala Gln Ser Val Met

        1475                1480                1485

Glu Gln Asn Thr Val Lys Glu Ile Pro Phe Leu Asn Glu Gln Thr Asn

    1490                1495                1500

Ser Lys Ile Glu Lys Thr Asp Asn Val Glu Val Thr Asp Gly Glu Asn

1505                1510                1515                1520

Lys Glu Ile Lys Val Lys Val Asp Asn Ile Ser Glu Ser Thr Asp Lys

                1525                1530                1535

Ser Ala Glu Ile Glu Thr Ser Val Val Gly Ser Ser Ser Ile Ser Ala

            1540                1545                1550

Gly Ser Leu Thr Ser Leu Ser Leu Arg Gly Lys Pro Pro Asp Val Ser

        1555                1560                1565

Thr Glu Ala Phe Leu Thr Asn Leu Ser Ile Gln Ser Lys Gln Glu Glu

    1570                1575                1580

Thr Val Glu Ser Lys Glu Lys Thr Leu Lys Arg Gln Leu Gln Glu Asp

1585                1590                1595                1600

Gln Glu Asn Asn Leu Gln Asp Asn Gln Thr Ser Asn Ser Ser Pro Cys

                1605                1610                1615

Arg Ser Asn Val Gly Lys Gly Asn Ile Asp Gly Asn Val Ser Cys Ser

            1620                1625                1630

Glu Asn Leu Val Ala Asn Thr Ala Arg Ser Pro Gln Phe Ile Asn Leu

        1635                1640                1645

Lys Arg Asp Pro Arg Gln Ala Ala Gly Arg Ser Gln Pro Val Thr Thr

    1650                1655                1660

Ser Glu Ser Lys Asp Gly Asp Ser Cys Arg Asn Gly Glu Lys His Met

1665                1670                1675                1680

Leu Pro Gly Leu Ser His Asn Lys Glu His Leu Thr Glu Gln Ile Asn

                1685                1690                1695

Val Glu Glu Lys Leu Cys Ser Ala Glu Lys Asn Ser Cys Val Gln Gln

            1700                1705                1710

Ser Asp Asn Leu Lys Val Ala Gln Asn Ser Pro Ser Val Glu Asn Ile

        1715                1720                1725

Gln Thr Ser Gln Ala Glu Gln Ala Lys Pro Leu Gln Glu Asp Ile Leu

    1730                1735                1740

Met Gln Asn Ile Glu Thr Val His Pro Phe Arg Arg Gly Ser Ala Val

1745                1750                1755                1760

Ala Thr Ser His Phe Glu Val Gly Asn Thr Cys Pro Ser Glu Phe Pro

                1765                1770                1775

Ser Lys Ser Ile Thr Phe Thr Ser Arg Ser Thr Ser Pro Arg Thr Ser

            1780                1785                1790

Thr Asn Phe Ser Pro Met Arg Pro Gln Gln Pro Asn Leu Gln His Leu

        1795                1800                1805

Lys Ser Ser Pro Pro Gly Phe Pro Phe Pro Gly Pro Pro Asn Phe Pro

    1810                1815                1820

Pro Gln Ser Met Phe Gly Phe Pro Pro His Leu Pro Pro Pro Leu Leu

1825                1830                1835                1840

Pro Pro Pro Gly Phe Gly Phe Ala Gln Asn Pro Met Val Pro Trp Pro

                1845                1850                1855

Pro Val Val His Leu Pro Gly Gln Pro Gln Arg Met Met Gly Pro Leu

            1860                1865                1870

Ser Gln Ala Ser Arg Tyr Ile Gly Pro Gln Asn Phe Tyr Gln Val Lys

        1875                1880                1885

Asp Ile Arg Arg Pro Glu Arg Arg His Ser Asp Pro Trp Gly Arg Gln

    1890                1895                1900

Asp Gln Gln Gln Leu Asp Arg Pro Phe Asn Arg Gly Lys Gly Asp Arg

1905                1910                1915                1920

Gln Arg Phe Tyr Ser Asp Ser His His Leu Lys Arg Glu Arg His Glu

1925                1930                1935

Lys Glu Trp Glu Gln Glu Ser Glu Arg His Arg Arg Arg Asp Arg Ser

        1940                1945                1950

Gln Asp Lys Asp Arg Asp Arg Lys Ser Arg Glu Glu Gly His Lys Asp

    1955                1960                1965

Lys Glu Arg Ala Arg Leu Ser His Gly Asp Arg Gly Thr Asp Gly Lys

1970                1975                1980

Ala Ser Arg Asp Ser Arg Asn Val Asp Lys Lys Pro Asp Lys Pro Lys

1985                1990                1995                2000

Ser Glu Asp Tyr Glu Lys Asp Lys Glu Arg Glu Lys Ser Lys His Arg

                2005                2010                2015

Glu Gly Glu Lys Asp Arg Asp Arg Tyr His Lys Asp Arg Asp His Thr

        2020                2025                2030

Asp Arg Thr Lys Ser Lys Arg

    2035

<210> 8

<211> 6948

<212> DNA

<213> Homo sapiens

<400> 8

ggggctgaaa gacacacaga agtcttcatg gatatagttg atacatttaa tcatttaatt      60

cctactgaac acttagatga tgccctattt ctaggatcca acctggagaa tgaagtctgt     120

gaggatttta gtgcaagtca aaatgtctta gaggactcgc tgaagaacat gctcagcgat     180

aaggatccta tgctaggatc tgcaagtaac cagttctgtt tgcctgtttt ggatagcaat     240

gatcccaatt tccagatgcc ttgttcaaca gttgttggtc ttgacgatat tatggatgaa     300

ggagttgtta aagaaagtgg caatgatacc attgatgaag aagaactgat tttacctaac 360
aggaacttaa gggacaaggt agaagaaaat tcagtgagat ctccaagaaa atcacctcgt 420
ttaatggcac aagaacaagt aagaagtttg cgacagagca ctattgccaa gcgttcaaat 480
gcagcaccat taagtaacac aaaaaaagca tctgggaaga ctgtatctac tgctaaagca 540
ggagtgaaac aaccagaaag gagtcaggtt aaagaagaag tatgtatgtc actgaaacct 600
gagtaccata aggagaatag aaggtgcagc cgaaatagcg gacaaattga agtggtacct 660
gaagtatcag tgtcttcaag tcattcttca gtgtcatctt gtcttgaaat gaaggatgaa 720
gatggattag attctaagca taagtgtaat aatccgggag aaatagatgt gccatctcat 780
gaattaaatt gttcacttct ttcagagact tgtgttacta ttggagaaaa gaaaaatgaa 840
gctttgatgg aatgtaaagc caagcctgtt ggtagtccat tgtttaagtt ttcagataaa 900
gaagaacatg aacaaaatga ttccatttca ggtaaaacgg gtgagactgt tgttgaagaa 960
atgatagcaa caagaaaagt tgaacaagat tcaaaggaga cagtaaaatt atcccatgaa 1020
gatgaccata ttcttgagga cgctggatct tctgatattt ctagtgatgc tgcttgtaca 1080
aatccaaata agacagaaaa cagccttgta ggtttgccta gttgtgtaga tgaagtgact 1140
gaatgtaatt tggaattgaa ggataccatg ggtattgctg ataaaactga gaacaccctt 1200
gaaagaaata aaattgaacc gttgggttat tgtgaagatg cggagtctaa taggcagttg 1260
gagagcactg agtttaataa atcaaactta gaggtggttg atactagtac ttttggaccg 1320
gaaagtaata tcttggaaaa tgctatttgt gatgtgcctg accaaaattc aaaacagttg 1380
aatgctatag aaagtactaa aatagagtcc catgaaacag caaaccttca ggatgacaga 1440
aacagccagt caagtagcgt ttcttactta gagtcaaaaa gtgtaaaatc caaacataca 1500
aaacctgtaa ttcattctaa gcaaaacatg accacagatg ctccgaagaa aattgttgca 1560
gcaaagtatg aagtaataca tagcaaaact aaagttaatg tcaaaagtgt gaaacgaaat 1620
actgatgtac cagaatctca gcaaaatttt cataggccag tcaaagtcag aaaaaaacaa 1680
attgataagg agccaaagat tcagagttgc aattctgggg ttaaatctgt gaaaaaccaa 1740
gctcattctg tactgaaaaa aacattacag gatcaaactt tagtacaaat tttcaagccc 1800
ttaactcatt ctttgagtga taagtcacac gctcatcctg gttgcttgaa agaacctcat 1860
catcctgcac aaactggaca tgtatcacat tctagccaga aacagtgtca taagcctcag 1920
caacaggccc cagcaatgaa aaccaatagt cacgtgaagg aagagcttga acacccaggc 1980

gttgagcatt ttaaggaaga ggataaactg aaactgaaaa aacctgagaa gaacctacaa 2040
ccccgccaaa gaagaagcag caaaagtttt tctttagatg agccaccatt gttcattcca 2100
gataacatag ctaccataag aagagaaggc tctgatcata gctcctcatt tgaaagcaaa 2160
tatatgtgga ctcccagcaa gcagtgtggg ttttgcaaaa aaccacatgg caacaggttt 2220
atggttggct gtgggagatg tgatgactgg tttcatggtg attgtgttgg gttaagtctt 2280
tctcaagcac agcagatggg cgaggaagac aaagaatatg tctgtgtaaa atgttgtgct 2340
gaagaagaca aaaagactga aatactagat ccagatactt tggaaaacca agctacagtt 2400
gaattccata gtggagataa aacaatggag tgtgaaaagc ttggattatc aaaacacaca 2460
acaaatgata gaaccaaata tatagatgat acagtgaagc acaaggtcaa aattttaaaa 2520
cgggagtctg gtgaaggcag aaattcatca gactgtagag ataatgaaat taaaaaatgg 2580
cagctagctc ctcttcgtaa gatgggacaa ccagttttac ctcggagatc ctcagaagaa 2640
aaaagtgaaa aaataccgaa agagtctaca actgttactt gcacaggaga aaaagcttca 2700
aaaccaggta ctcatgagaa gcaagagatg aaaaagaaga aagttgaaaa aggagtgctt 2760
aatgtacatc ctgctgcttc tgcttccaag ccttctgcag atcagatcag gcaaagtgtc 2820
agacattctc tcaaagacat tcttatgaag agacttacag actcaaattt gaaggtacca 2880
gaggaaaagg cagcaaaagt tgccacaaaa attgagaaag agcttttctc tttttttcgg 2940
gacacagatg ctaaatataa gaacaaatat agaagtttga tgtttaattt gaaagatcct 3000
aaaaacaata tattatttaa aaaagtactg aaaggagaag taactcctga tcatcttatc 3060
agaatgagtc cagaagaact agcttctaaa gagttagctg cttggagacg aagagaaaac 3120
agacatacca tagaaatgat tgagaaagag cagagagaag tggaacgacg gccaatcacc 3180
aaaataactc ataaaggtga aatagaaatt gagagtgatg ccccaatgaa agaacaggaa 3240
gcagccatgg agattcagga accagccgcc aataagtcat tggagaagcc agaaggatct 3300
gaaaaacaaa aagaggaggt tgactctatg tctaaagata ccactagtca acacagacag 3360
catctttttg atctcaactg caaaatctgc ataggtcgaa tggcaccacc tgtagatgat 3420
ctttctccaa aaaaagtaaa agttgttgta ggagtagctc gcaaacattc agacaatgaa 3480
gcagaaagta tagcagatgc attatcttca acctcaaata ttttggcttc tgaattcttt 3540
gaggaggaga aacaggagtc tccaaagtca acgttctctc ctgctccacg tccagagatg 3600
cctggaactg ttgaagttga gtctaccttt ctggctcgat tgaacttcat ctggaaaggt 3660

tttatcaaca tgccttctgt ggcaaaattt gttaccaaag cctatccagt atctggctcc 3720
ccagaatacc tgacagagga cctaccagat agtattcaag taggtggcag gatatcacct 3780
cagacagttt gggattatgt ggaaaaaata aaagcatcag gaaccaagga aatttgtgtg 3840
gttcgcttca caccagtaac tgaagaagat caaatttctt atactttgct ctttgcatac 3900
ttcagtagca gaaagcgcta tggagtagct gctaacaaca tgaagcaggt taaagatatg 3960
taccttattc ctttgggtgc cacagataaa attccacacc ctcttgtgcc ttttgatgga 4020
cctgggcttg aactgcatag acctaatcta ttgttgggct taattattcg tcagaaactg 4080
aagcgacagc acagtgcctg tgctagtact agtcatatag ctgagactcc tgaaagtgca 4140
ccaccaatag cattgccacc tgataaaaaa agtaaaatag aagtttctac agaagaagca 4200
ccagaggaag aaaatgactt ttttaattct tttacaactg tattacacaa gcagagaaat 4260
aaacctcagc agaatcttca ggaagacctt ccaacagcag ttgaaccttt aatggaagtc 4320
accaaacagg agccaccaaa acctttaaga tttcttcctg gcgtgttgat tggctgggag 4380
aatcaaccta ctactctgga attagcaaat aaacctcttc ctgtggatga tatacttcaa 4440
agccttttgg gcaccactgg tcaagtatat gaccaggccc agtcagtgat ggaacaaaac 4500
actgttaaag aaattccatt tttaaatgag cagaccaact caaaaataga gaaaacagat 4560
aatgtggaag taactgatgg tgaaaacaag gagataaaag ttaaagtaga taatatttca 4620
gaatctacag ataagtcagc agaaatagaa acatcagtag tagggtcctc ttccatttct 4680
gcagggtctt tgacgagtct tagtctcaga ggtaagccac cagatgtttc tacagaagca 4740
tttttaacaa atttatcaat tcagtcaaaa caagaggaaa ctgtggagag taaagagaaa 4800
acattaaaaa gacagcttca ggaagatcaa gagaataatt tgcaagataa ccagacttca 4860
aatagttctc catgcagatc taatgtagga aaaggaaaca tagatggtaa tgtgagctgt 4920
agtgaaaacc ttgttgctaa tacagcgagg tctccacagt ttatcaacct gaaaagggat 4980
cctaggcaag cagcaggacg aagtcagcct gtaactactt cagaaagcaa agatggagat 5040
agttgccgga atggagaaaa acacatgctg cctggcctgt cacacaacaa ggagcactta 5100
acagaacaaa tcaatgtaga ggaaaagttg tgttctgcag agaaaaactc gtgtgttcag 5160
cagagtgaca atttaaaagt tgcacaaaac tcaccatcag tagaaaacat acagacttct 5220
caagcagaac aagcaaaacc cttacaggag gatattttaa tgcaaaatat tgaaactgtg 5280
cacccatttc gaagaggatc agcagtagcg acatctcatt ttgaagttgg aaacacatgt 5340

ccatcagaat ttccttctaa aagcatcacc tttacttcca gaagcaccag ccccagaaca 5400
agtacaaact tttcacccat gaggccacag cagcccaacc ttcagcatct caagtctagc 5460
ccacctggat ttccatttcc agggcctcct aattttcccc cacaaagcat gtttggattt 5520
ccaccacatt tgccacctcc attacttccc cctccaggct ttggctttgc tcaaaatccc 5580
atggttccct ggccacctgt tgttcatctc ccaggtcagc cacagcgtat gatgggtcct 5640
ctctcacaag catcaaggta tataggcccg cagaattttt accaggttaa agacattcgg 5700
aggccagaaa ggcgccatag tgacccttgg ggtaggcaag accaacagca actggatagg 5760
ccatttaata ggggtaaagg ggaccgccag agattttata gtgattcaca ccatttgaaa 5820
agagagcgac atgaaaagga atgggagcaa gaatctgaaa ggcatagacg cagagacaga 5880
agccaagaca aggacagaga cagaaaaagc agggaggaag ggcacaaaga taaagagagg 5940
gcacggttat cacatggtga tcgaggaaca gatggaaaag caagcagaga tagtaggaat 6000
gtagacaaga agccagataa acctaaaagt gaagactatg agaaggacaa agaacgagag 6060
aaaagtaaac acagagaagg agaaaaggac agggataggt accacaaaga tagggaccac 6120
actgacagaa ctaaaagcaa aaggtaaaat ttgcaggctg cttcaggatt acatttaaat 6180
aactgttaaa atgttgtatc ttgtaaacaa aagaaagatt gcctgctagg attgtgccat 6240
ctttaaaatt tttactattg gtcatttgca gaacagtaaa ttctgtgtgt tggtacagag 6300
tgctctgtac cagtgctcat catcccttct tcataccaac ggtccctagt tataggaatt 6360
taatattttt aaaagtttta cattgctgta tattcaaaga tttgttttat taatatgcaa 6420
taaaggctta gaaattttag ttttattcct taattggtaa atatggttaa ctatggaata 6480
tatttacttc ctctagtgaa tgtcctttat ataatgacta atttgggagt aatgtgtgct 6540
ctgtaagttt gttttaaatt gcactgtttt taaagaaact gtagaggagc aacaaaaatc 6600
caagcaactt cataatcaga ttatgctaat catttagttg agcagttttt gaccaagaat 6660
cagaagccca aggggtacat ttattgcttt aatctgcact cattgaagtc atttattacc 6720
atatactaca gctttgtggt aggccattat tttcattttc atttttggct cttcagaaac 6780
ttgaatactt aagcttgtac atgatcttgt gttttgctat cctttttact gtaaaatgta 6840
aatattttaa gggatatttt gattctaaat atgataaaat aatttctcac ctattttgtg 6900
tgtgtgactt gaaattcagt agtaaaagaa tttcttcttt aaagcttt 6948

<210> 9

<211> 20

<212> DNA

<213> Artificial sequence

<220>

<223> Designed oligonucleotide primer for PCR

<400> 9

cccaatttcc agatgccttg        20

<210> 10

<211> 20

<212> DNA

<213> Artificial sequence

<220>

<223> Designed oligonucleotide primer for PCR

<400> 10

gcatgcatgt ttgggatcaa        20